(19) Europäisches Patentamt
European Patent Office
Office européen des brevets

(11) Veröffentlichungsnummer: **0 259 793 B1**

(12) # EUROPÄISCHE PATENTSCHRIFT

(45) Veröffentlichungstag der Patentschrift: **01.07.92**

(51) Int. Cl.⁵: **C07D 217/24**, C07D 217/04, C07D 223/16, C07D 491/056, C07D 209/46, A61K 31/47, A61K 31/55, A61K 31/40

(21) Anmeldenummer: **87112920.1**

(22) Anmeldetag: **04.09.87**

Die Akte enthält technische Angaben, die nach dem Eingang der Anmeldung eingereicht wurden und die nicht in dieser Patentschrift enthalten sind.

(54) **Neue Naphthylderivate, diese Verbindungen enthaltende Arzneimittel und Verfahren zu ihrer Herstellung.**

(30) Priorität: **12.09.86 DE 3631013**

(43) Veröffentlichungstag der Anmeldung:
**16.03.88 Patentblatt 88/11**

(45) Bekanntmachung des Hinweises auf die Patenterteilung:
**01.07.92 Patentblatt 92/27**

(84) Benannte Vertragsstaaten:
**AT BE CH DE ES FR GB GR IT LI LU NL SE**

(56) Entgegenhaltungen:
**EP-A- 0 109 636**
**EP-A- 0 161 599**
**CH-A- 622 781**
**DD-A- 224 318**
**GB-A- 1 548 844**

(73) Patentinhaber: **Dr. Karl Thomae GmbH**
**Postfach 1755**
**W-7950 Biberach (Riss)(DE)**

(72) Erfinder: **Heider, Joachim, Dr., Dipl. Chem.**
**Am Hang 3**
**W-7951 Warthausen 1(DE)**
Erfinder: **Psiorz, Manfred, Dr., Dipl. Chem.**
**Riedlinger Strasse 35**
**W-7950 Biberach 1(DE)**
Erfinder: **Bomhard, Andreas, Dr., Dipl. Chem.**
**Dinglingerstrasse 9**
**W-7950 Biberach 1(DE)**
Erfinder: **Hauel, Norbert, Dr., Dipl. Chem.**
**Sresemannstrasse 40**
**W-7950 Biberach-Bachlangen(DE)**
Erfinder: **Narr, Berthold, Dr., Dipl. Chem.**
**Obere Au 5**
**W-7950 Biberach 1(DE)**
Erfinder: **Noll, Klaus, Dr., Dipl. Chem.**
**Im Schönblick 3**
**W-7951 Warthausen(DE)**
Erfinder: **Lillie, Christian, Dr.**
**Hansi-Niese-Weg 12**
**A-1130 Wien(AT)**

EP 0 259 793 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Kobinger, Walter, Prof. Dr.**
**Belghofergasse 27**
**A-1121 Wien(AT)**
Erfinder: **Dämmgen, Jürgen, Dr.**
**Eichweg 7**
**W-7951 Sulmigen(DE)**

## Beschreibung

In der britischen Patentschrift 1 548 844 werden Isochinolin-1-one und 1,2,3,4-Tetrahydro-5H-2-benzazepin-1-one, in der EP-A-0.109.636 3-[(Phenylalkyl)aminoalkyl]-2H-3-benzazepine und in der EP-A-0.161.599 u.a. 3-[(Phenyloxyalkyl)aminoalkyl]-2H-3-benzazepine und deren physiologisch verträgliche Säureadditionssalze beschrieben, welche wertvolle pharmakologische Eigenschaften aufweisen, nämlich neben einer milden blutdrucksenkenden Wirkung insbesondere eine selektive herzfrequenzsenkende Wirkung.

Überraschenderweise wurde nun gefunden, daß die neuen Naphthylderivate der allgemeinen Formel

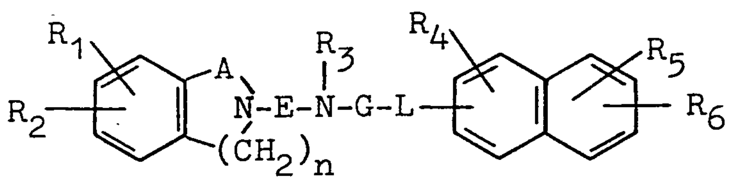

(I)

und, falls diese ein optisch aktives Kohlenstoffatom enthalten, deren Enantiomeren sowie deren Säureadditionssalze, insbesondere deren physiologisch verträgliche Säureadditionssalze mit anorganischen oder organischen Säuren, noch wertvollere pharmakologische Eigenschaften aufweisen, insbesondere eine langanhaltende herzfrequenzsenkende Wirkung und eine herabsetzende Wirkung auf den $O_2$-Bedarf des Herzens.

Gegenstand der vorliegenden Erfindung sind somit die neuen Naphthylderivate der obigen allgemeinen Formel I, deren Enantiomeren, deren Säureadditionssalze, insbesondere für die pharmazeutische Verwendung deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel I bedeuten

n die Zahl 1 oder 2,

A eine -CO-,

$$-\underset{x}{C}H_2CO-$$

oder -COCO-Gruppe, wobei das mit x gekennzeichnete Atom mit dem Phenylkern verknüpft ist,

E eine gegebenenfalls durch eine Alkylgruppe mit 1 bis 3 Kohlenstoffatomen substituierte geradkettige Alkylengruppe mit 2 bis 4 Kohlenstoffatomen,

G eine gegebenenfalls durch eine Alkylgruppe mit 1 bis 3 Kohlenstoffatomen substituierte geradkettige Alkylengruppe mit 1 bis 5 Kohlenstoffatomen,

L eine Bindung oder auch ein Sauerstoffatom, wenn G eine gegebenenfalls durch eine Alkylgruppe mit 1 bis 3 Kohlenstoffatomen substituierte geradkettige Alkylengruppe mit 2 bis 5 Kohlenstoffatomen darstellt,

$R_1$ und $R_2$, die gleich oder verschieden sein können, Alkyl- oder Alkoxygruppen mit jeweils 1 bis 3 Kohlenstoffatomen in jedem Alkylteil oder $R_1$ und $R_2$ zusammen eine Alkylendioxygruppe mit 1 oder 2 Kohlenstoffatomen,

$R_3$ ein Wasserstoffatom, eine Alkylgruppe mit 1 bis 3 Kohlenstoffatomen oder eine Allylgruppe,

$R_4$, $R_5$ und $R_6$, die gleich oder verschieden sein können, Wasserstoffatome, Alkyl- oder Alkoxygruppen mit jeweils 1 bis 3 Kohlenstoffatomen in jedem Alkylteil.

Für die bei der Definition der Reste eingangs erwähnten Bedeutungen kommt beispielsweise

für A und n die eingangs erwähnten Bedeutungen,

für $R_1$ die der Methyl-, Ethyl-, n-Propyl-, Isopropyl-, Methoxy-, Ethoxy-, n-Propoxy- oder Isopropoxygruppe,

für $R_2$ die der Methyl-, Ethyl-, n-Propyl-, Isopropyl-, Methoxy-, Ethoxy-, n-Propoxy- oder Isopropoxygruppe oder zusammen mit $R_1$ die der Methylendioxy- oder Ethylendioxygruppe,

für $R_3$ die des Wasserstoffatoms, der Methyl-, Ethyl-, n-Propyl-, Isopropyl- oder Allylgruppe,

für $R_4$ die des Wasserstoffatoms, der Methyl-, Ethyl-, n-Propyl-, Isopropyl-, Methoxy-, Ethoxy-, n-Propoxy- oder Isopropoxygruppe,

für $R_5$ die des Wasserstoffatoms, der Methyl-, Ethyl-, n-Propyl-, Isopropyl-, Methoxy-, Ethoxy-, n-Propoxy- oder Isopropoxygruppe,

für $R_6$ die des Wasserstoffatoms, der Methyl-, Ethyl-, n-Propyl-, Isopropyl-, Methoxy-, Ethoxy-, n-Propoxy- oder Isopropoxygruppe,
für E die der Äthylen-, n-Propylen-, n-Butylen-, 1-Methylethylen-, 1-Ethyl-ethylen-, 2-Methyl-ethylen-, 2-Ethyl-ethylen-, 1-n-Propyl-ethylen-, 1-Methyl-n-propylen, 2-Methyl-n-propylen-,3-Methyl-n-propylen-, 1-Ethyl-n-propylen-, 2-n-Propyl-n-propylen- oder 3-Ethyl-n-propylengruppe und
für -G-L- die der Methylen-, Ethyliden-, n-Propyliden-, n-Butyliden-, 2-Methyl-propyliden-, Ethylen-, 1-Methyl-ethylen-, 2-Ethyl-ethylen-, 1-Propyl-ethylen-, 2-Methyl-ethylen-, 1-Ethyl-ethylen-, n-Propylen-, n-Butylen-, n-Pentylen-, 1-Methyl-n-propylen-, 1-Methyl-butylen-, 1-Methyl-n-pentylen, 1-Ethyl-n-propylen-, 2-Ethyl-n-propylen-, 1-Methyl-n-butylen-,Ethylenoxy-, n-Propylenoxy-, n-Butylenoxy- oder n-Pentylenoxygruppe in Betracht.

Erfindungsgemäß fallen somit folgende Verbindungen unter die vorstehend genannte allgemeine Formel I:
2-[N-Methyl-N-((2-methyl-naphth-1-yl)-eth-2-yl)-amino-prop-3-yl]-6,7-dimethoxy-1,2,3,4-tetrahydro-isochinolin-1-on
2-[N-Methyl-N-((naphth-2-yl)-prop-2-yl)-amino-prop-3-yl]-6,7-dimethoxy-1,2,3,4-tetrahydro-isochinolin-1-on
2-[N-Methyl-N-((5-methyl-6-methoxy-naphth-2-yl)-eth-2-yl)-amino-prop-3-yl]-6,7-dimethoxy-1,2,3,4-tetrahydro-isochinolin-1-on
3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on
2-[N-Methyl-N-((naphth-2-oxy)-but-4-yl)-amino-prop-3-yl]-7,8-dimethoxy-1,2,3,4-tetrahydro-isochinolin-1-on
2-[N-Methyl-N-((2-methyl-naphth-1-yl)-methyl)-amino-prop-3-yl]-6,7-dimethoxy-1,2,3,4-tetrahydro-isochinolin-1-on
3-[N-Methyl-N-((naphth-1-yl)-eth-2-yl)-amino-prop-3-yl]-7,8-dimethoxy-1,3,4,5-tetrahydro-2H-3-benzazepin-2-on
2-[N-Methyl-N-((6-methoxy-naphth-2-yl)-eth-2-yl)-amino-prop-3-yl]-6,7-methylendioxy-1,2,3,4-tetrahydro-isochinolin-1-on
2-[N-Methyl-N-((naphth-1-oxy)-eth-2-yl)-amino-prop-3-yl]-5,6-dimethoxy-phthalimidin
3-[N-Methyl-N-((naphth-2-yl)-eth-2-yl)-amino-prop-3-yl]-6,7-dimethoxy-1,2,3,4-tetrahydro-isochinolin-1-on
2-[N-Methyl-N-((2-methyl-naphth-1-yl)-eth-2-yl)-prop-3-yl]-6,7-methylendioxy-1,2,3,4-tetrahydro-isochinolin-1-on
2-[N-Methyl-N-((naphth-2-yl)-prop-2-yl)-amino-prop-3-yl]-6,7-dimethyl-1,2,3,4-tetrahydro-isochinolin-1-on
2-[N-Methyl-N-((naphth-2-yl)-eth-2-yl)-amino-prop-3-yl]-6,7-methylendioxy-1,2,3,4-tetrahydro-isochinolin-1-on
2-[N-Methyl-N-((naphth-2-yl)-prop-3-yl)-amino-prop-3-yl]-6,7-dimethoxy-1,2,3,4-tetrahydro-isochinolin-1-on
2-[N-Methyl-N-((naphth-2-yl)-prop-3-yl)-amino-prop-3-yl]-6,7-methylendioxy-1,2,3,4-tetrahydro-isochinolin-1-on
2-[N-Methyl-N-((naphth-2-yl)-prop-3-yl)-amino-prop-3-yl]-6,7-dimethyl-1,2,3,4-tetrahydro-isochinolin-1-on
2-[N-Methyl-N-((5-methyl-6-methoxy-naphth-2-yl)-eth-2-yl)-amino-prop-3-yl]-6,7-methylendioxy-1,2,3,4-tetrahydro-isochinolin-1-on
2-[N-Methyl-N-((naphth-2-oxy)-but-4-yl)-amino-prop-3-yl]-6,7-methylendioxy-isochinolin-1-on
2-[N-Methyl-N-((6-methoxy-naphth-2-yl)-eth-2-yl)-amino-prop-3-yl]-6,7-dimethyl-1,2,3,4-tetrahydro-isochinolin-1-on
2-[N-Methyl-N-((2-methyl-naphth-1-yl)-methyl)-amino-prop-3-yl]-6,7-dimethyl-1,2,3,4-tetrahydro-isochinolin-1-on
3-[N-Methyl-N-((6-methoxy-5-methyl-naphth-2-yl)-eth-2-yl)-amino-prop-3-yl]-7,8-dimethoxy-1,3,4,5-tetrahydro-2H]-benzazepin-2-on
3-[N-Methyl-N-((2-methyl-naphth-1-yl)-methyl)-amino-prop-3-yl]-7,8-dimethoxy-1,3,4,5-tetrahydro-2H]-benzazepin-2-on
3-[N-Methyl-N-((2-naphth-1-yl)-eth-2-yl)-amino-prop-3-yl]-7,8-dimethoxy-1,3,4,5-tetrahydro-2H-benzazepin-2-on
3-[N-Methyl-N-((naphth-1-yl)-prop-2-yl)-amino-prop-3-yl]-7,8-dimethoxy-1,3,4,5-tetrahydro-2H-3-benzazepin-2-on
3-[N-Methyl-N-((naphth-2-oxy)-but-4-yl)-amino-prop-3-yl]-7,8-dimethoxy-1,3,4,5-tetrahydro-2H-3-benzazepin-2-on
3-[N-Methyl-N-((2-methyl-naphth-1-yl)-eth-2-yl)-amino-prop-3-yl]-7,8-dimethoxy-1,3,4,5-tetrahydro-2H-3-benzazepin-2-on
3-[N-Methyl-N-((naphth-1-yl)-eth-2-yl)-amino-prop-3-yl]-7,8-dimethoxy-1,3,4,5-tetrahydro-2H-3-benzazepin-1,2-dion
2-[N-Methyl-N-((naphth-2-yl)-methyl)-amino-prop-3-yl]-5,6-dimethoxy-phthalimidin

2-[N-Methyl-N-((naphth-2-yl-oxy)-but-4-yl)-amino-prop-3-yl]-5,6-dimethoxy-phthalimidin
2-[N-Methyl-N-((naphth-1-yl)-eth-2-yl)-amino-prop-3-yl]-5,6-dimethoxy-phthalimidin
2-[N-Methyl-N-((5-methyl-6-methoxy-naphth-2-yl)-eth-2-yl)-amino-prop-3-yl]-5,6-dimethoxy-phthalimidin
2-[N-Methyl-N-((2-methyl-naphth-1-yl)-methyl)-amino-prop-3-yl]-5,6-dimethoxy-phthalimidin
3-[N-Methyl-N-((naphth-2-yl)-eth-2-yl)-amino-prop-3-yl)-7,8-dimethoxy-1,3,4,5-tetrahydro-2H-3-benzazepin-2-on
2-[N-Methyl-N-((naphth-2-yl)-eth-2-yl)-amino-prop-3-yl]-6,7-dimethyl-1,2,3,4-tetrahydro-isochinolin-1-on
2-[N-Methyl-N-((2-methyl-naphth-1-yl)-eth-2-yl)-amino-prop-3-yl]-6,7-dimethyl-1,2,3,4-tetrahydro-isochinolin-1-on
2-[N-Methyl-N-((naphth-2-yl)-prop-2-yl)-amino-prop-3-yl]-6,7-methylendioxy-1,2,3,4-tetrahydro-isochinolin-1-on
2-[N-Methyl-N-((5-methyl-6-methoxy-naphth-2-yl)-eth-2-yl)-amino-prop-3-yl]-6,7-dimethyl-1,2,3,4-tetrahydro-isochinolin-1-on
2-[N-Methyl-N-((6-methoxy-naphth-2-yl)-eth-2-yl)-amino-prop-3-yl]-6,7-dimethoxy-1,2,3,4-tetrahydro-isochinolin-1-on
2-[N-Methyl-N-((2-methyl-naphth-1-yl)-methyl)-amino-prop-3-yl]-6,7-methylendioxy-1,2,3,4-tetrahydro-isochinolin-1-on
2-[N-Methyl-N-((naphth-2-oxy)-but-4-yl)-amino-prop-3-yl]-6,7-dimethyl-1,2,3,4-tetrahydro-isochinolin-1-on
2-[N-Methyl-N-((naphth-1-yl)-prop-3-yl)-amino-prop-3-yl]-6,7-dimethoxy-1,2,3,4-tetrahydro-isochinolin-1-on
2-[N-Methyl-N-((naphth-1-yl)-prop-3-yl)-amino-prop-3-yl]-6,7-methylendioxy-1,2,3,4-tetrahydro-isochinolin-1-on
2-[N-Methyl-N-((naphth-1-yl)-prop-3-yl)-amino-prop-3-yl]-6,7-dimethyl-1,2,3,4-tetrahydro-isochinolin-1-on
2-[N-Methyl-N-((naphth-1-yl)-eth-2-yl)-amino-prop-3-yl]-6,7-dimethoxy-1,2,3,4-tetrahydro-isochinolin-1-on
2-[N-Methyl-N-((naphth-1-yl)-eth-2-yl)-amino-prop-3-yl]-6,7-methylendioxy-1,2,3,4-tetrahydro-isochinolin-1-on
2-[N-Methyl-N-((naphth-1-yl)-eth-2-yl)-amino-prop-3-yl]-6,7-dimethyl-1,2,3,4-tetrahydro-isochinolin-1-on
3-[N-Methyl-N-((6-methoxy-5-methyl-naphth-2-yl)-eth-2-yl)-amino-prop-3-yl]-7,8-dimethyl-1,3,4,5-tetrahydro-2H-3-benzazepin-2-on
3-[N-Methyl-N-((6-methoxy-naphth-2-yl)-eth-2-yl)-amino-prop-3-yl]-7,8-methylendioxy-1,3,4,5-tetrahydro-2H-3-benzazepin-2-on
3-[N-Methyl-N-((6-methoxy-naphth-2-yl)-prop-3-yl)-amino-prop-3-yl]-7,8-methylendioxy-1,3,4,5-tetrahydro-2H-3-benzazepin-2-on
2-[N-Methyl-N-((2-methyl-naphth-1-yl)-eth-2-yl)-amino-eth-2-yl]-7,8-dimethyl-1,3,4,5-tetrahydro-2H-3-benzazepin-2-on
3-[N-Methyl-N-((naphth-2-oxy)-but-4-yl)-amino-prop-3-yl]-7,8-methylendioxy-1,3,4,5-tetrahydro-2H-3-benzazepin-2-on
3-[N-Methyl-N-((2-methyl-naphth-1-yl)-methyl)-amino-prop-3-yl]-7,8-dimethyl-1,3,4,5-tetrahydro-2H-3-benzazepin-2-on
3-[N-Methyl-N-((naphth-2-oxy)-but-4-yl)-amino-prop-3-yl]-7,8-dimethyl-1,3,4,5-tetrahydro-2H-3-benzazepin-2-on
2-[N-Methyl-N-((6-methoxy-5-methyl-naphth-2-yl)-eth-2-yl)-amino-eth-2-yl]-7,8-methylendioxy-1,3,4,5-tetrahydro-2H-3-benzazepin-2-on
3-[N-Methyl-N-((6-methoxy-naphth-2-yl)-eth-2-yl)-amino-prop-3-yl]-7,8-dimethyl-1,3,4,5-tetrahydro-2H-3-benzazepin-2-on
2-[N-Methyl-N-((naphth-1-yl)-eth-2-yl)-amino-eth-2-yl]-7,8-methylendioxy-1,3,4,5-tetrahydro-2H-3-benzazepin-2-on
3-[N-Methyl-N-((2-methyl-naphth-1-yl)-eth-2-yl)-amino-prop-3-yl]-7,8-methylendioxy-1,3,4,5-tetrahydro-2H-3-benzazepin-2-on
2-[N-Methyl-N-((6-methoxy-naphth-2-yl)-eth-2-yl)-amino-eth-2-yl]-5-methyl-phthalimidin
2-[N-Methyl-N-((5-methyl-6-methoxy-naphth-2-yl)-eth-2-yl)-amino-eth-2-yl]-5,6-dimethyl-phthalimidin
2-[N-Methyl-N-((6-methoxy-naphth-2-yl)-eth-2-yl)-amino-eth-2-yl]-5,6-methylendioxy-phthalimidin
2-[N-Methyl-N-((5-methyl-6-methoxy-naphth-2-yl)-eth-2-yl)-amino-eth-2-yl]-5,6-methylendioxy-phthalimidin
2-[N-Methyl-N-((5-methyl-6-methoxy-naphth-2-yl)-eth-2-yl)-amino-eth-2-yl]-5,6-methylendioxy-phthalimidin
2-[N-Methyl-N-((5-methyl-6-methoxy-naphth-2-yl)-eth-2-yl)-amino-prop-3-yl]-5,6-dimethyl-phthalimidin
2-[N-Methyl-N-((naphth-1-yl)-eth-2-yl)-amino-prop-3-yl]-5,6-methylendioxy-phthalimidin
2-[N-Methyl-N-((2-methyl-naphth-1-yl)-prop-3-yl)-amino-eth-2-yl]-5,6-dimethyl-phthalimidin
2-[N-Methyl-N-((6-methoxy-naphth-2-yl)-but-4-yl)-amino-prop-3-yl]-5,6-dimethyl-phthalimidin
2-[N-Methyl-N-((2-methyl-naphth-1-yl)-but-4-yl)-amino-prop-3-yl]-5,6-dimethyl-phthalimidin
2-[N-Methyl-N-((6-methoxy-naphth-2-yl)-but-4-yl)-amino-prop-3-yl]-5,6-methylendioxy-phthalimidin

2-[N-Methyl-N-((5-methyl-6-methoxy-naphth-2-yl)-oxybut-4-yl)-amino-prop-3-yl]-5,6-methylendioxy-phthalimidin
2-[N-Methyl-N-((6-methoxy-naphth-2-yl)-oxyprop-3-yl)-amino-eth-2-yl]-5,6-dimethoxy-phthalimidin
2-[N-Methyl-N-((6-methoxy-naphth-2-yl)-oxyprop-3-yl)-amino-prop-3-yl]-5,6-dimethoxy-phthalimidin
2-[N-Methyl-N-((naphth-1-yl)-oxyprop-3-yl)-amino-prop-1-yl]-5,6-methylendioxy-phthalimidin
2-[N-Methyl-N-((naphth-1-yl)-pent-5-yl)-amino-eth-2-yl]-5,6-dimethyl-phthalimidin
2-[N-Methyl-N-((2-methyl-naphth-1-yl)-pent-5-yl)-amino-eth-2-yl]-5,6-methylendioxy-phthalimidin
2-[N-Methyl-N-((2-methyl-naphth-1-yl)-pent-5-yl)-amino-prop-3-yl]-5,6-dimethyl-phthalimidin
2-[N-Methyl-N-((5-methyl-6-methoxy-naphth-2-yl)-pent-5-yl)-amino-prop-3-yl]-5,6-methylendioxy-phthalimidin

Bevorzugte Verbindungen der obigen allgemeinen Formel I sind jedoch diejenigen, in denen
A, L und n wie eingangs definiert sind,
E eine Ethylen- oder n-Propylengruppe,
G eine gegebenenfalls durch eine Methylgruppe substituierte geradkettige Alkylengruppe mit 1 bis 4 Kohlenstoffatomen,
$R_1$ eine Methyl- oder Methoxygruppe,
$R_2$ eine Methyl- oder Methoxygruppe oder $R_1$ und $R_2$ zusammen eine Methylendioxygruppe,
$R_3$ eine Methylgruppe,
$R_4$ ein Wasserstoffatom oder Methylgruppe,
$R_5$ ein Wasserstoffatom, eine Methyl- oder Methoxygruppe,
$R_6$ ein Wasserstoffatom oder eine Methoxygruppe bedeuten, deren Enantiomere und deren Säureadditionssalze.

Besonders bevorzugte Verbindungen der allgemeinen Formel I sind jedoch diejenigen, in denen
n die Zahl 1 oder 2,
A eine -CO- oder $-CH_2CO-$Gruppe,
E eine n-Propylengruppe,
G eine Ethylengruppe,
L eine Bindung,
$R_1$ und $R_2$ jeweils eine Methoxygruppe oder zusammen eine Methylendioxygruppe,
$R_3$ eine Methylgruppe,
$R_4$ ein Wasserstoffatom,
$R_5$ ein Wasserstoffatom, eine Methyl- oder Methoxygruppe und
$R_6$ ein Wasserstoffatom oder eine Methoxygruppe bedeuten, und deren Säureadditionssalze.

Erfindungsgemäß erhält man die neuen Verbindungen nach folgenden Verfahren:

a) Umsetzung einer Verbindung der allgemeinen Formel

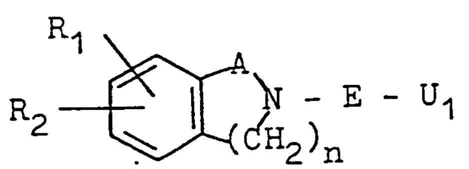

(II)

mit einer Verbindung der allgemeinen Formel

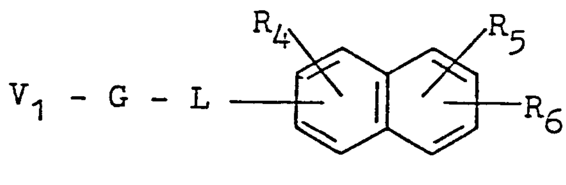

(III)

in denen
$R_1$, $R_2$, $R_4$ bis $R_6$, A, E, G, L und n wie eingangs definiert sind,
einer der Reste $U_1$ oder $V_1$ eine $R_3$-NH-Gruppe, wobei $R_3$ wie eingangs definiert ist und
der andere der Reste $U_1$ oder $V_1$ eine nukleophile Austrittsgruppe wie ein Halogenatom oder eine Sulfonyloxygruppe, z.B. ein Chlor-, Brom- oder Jodatom, die Methansulfonyloxy-, Benzolsulfonyloxy-, p-Toluolsulfonyloxy- oder Ethoxysulfonyloxygruppe, darstellt.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150 °C, vorzugsweise bei Temperaturen zwischen 50 und 120 °C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formeln II oder III durchgeführt.

b.) Umsetzung einer Verbindung der allgemeinen Formel

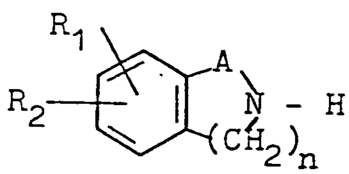

(IV)

in der

$R_1$, $R_2$, A und n wie eingangs definiert sind, mit einer Verbindung der allgemeinen Formel

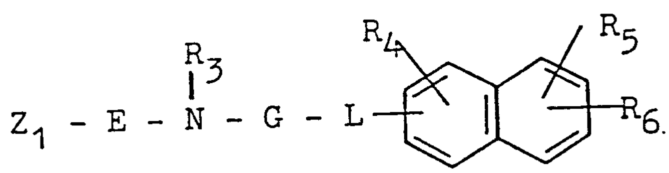

(V)

in der

$R_3$ bis $R_6$, E, G und L wie eingangs definiert sind und

$Z_1$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt.

Die Umsetzung wird zweckmäßigerweise in einem Lösungsmittel oder Lösungsmittelgemisch wie Methylformamid, Dimethylformamid, Dimethylsulfoxid, Benzol, Chlorbenzol, Tetrahydrofuran, Benzol/Tetrahydrofuran oder Dioxan in Gegenwart eines säurebindenden Mittels, z.B. eines Alkoholats wie Kaliumtert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150 °C, vorzugsweise bei Temperaturen zwischen 0 und 50 °C, durchgeführt.

c) Reduktive Aminierung einer Verbindung der allgemeinen Formel

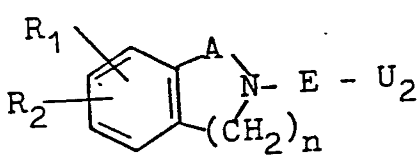

(VI)

in Gegenwart einer Verbindung der allgemeinen Formel

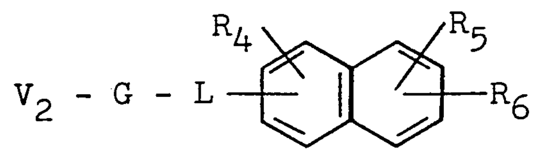

(VII)

in denen
$R_1$, $R_2$, $R_4$ bis $R_6$, A, E, G, L und n wie eingangs definiert sind,
einer der Reste $U_2$ oder $V_2$ eine $R_3$-NH-Gruppe, wobei
$R_3$ wie eingangs definiert ist, und
der andere der Reste $U_2$ oder $V_2$ zusammen mit einem Wasserstoffatom des benachbarten Kohlenstoffatoms des Restes G oder E, wobei E und G jeweils wie eingangs definiert sind, ein Sauerstoffatom bedeutet.

Die Reduktion wird in einem geeigneten Lösungsmittel wie Methanol, Ethanol, Diethylether, Tetrahydrofuran, Dioxan, Essigsäureethylester oder Ethanol/Essigsäureethylester mit einem Metallhydrid wie Lithiumaluminiumhydrid, Diboran, Natriumcyanborhydrid oder Boran/Dimethylsulfid, vorzugsweise jedoch mit Natriumborhydrid, oder mit Wasserstoff in Gegenwart eines Hydrierungskatalysators wie Platin, Palladium/Kohle oder Raney-Nickel bei einem Wasserstoffdruck von 1 bis 5 bar oder mit Hydrazin in Gegenwart eines Hydrierungskatalysators wie Platin, Palladium/Kohle oder Raney-Nickel, bei Temperaturen zwischen 0 und 50 ° C, vorzugsweise bei Raumtemperatur, durchgeführt. - Bei der Reduktion mit einem komplexen Metallhydrid wie Lithiumaluminiumhydrid, Diboran oder Boran/Dimethylsulfid, kann eine im Rest A vorhandene Carbonylfunktion zu einer Methylengruppe oder bei der katalytischen Hydrierung eine im Rest A vorhandenen Doppelbindung mitreduziert werden.

d) Reduktion eines Säureamids der allgemeinen Formel

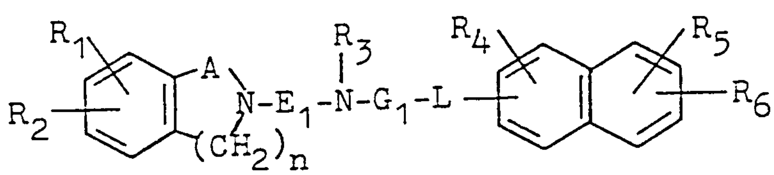

(VIII)

in der
$R_1$ bis $R_6$, A, L und n wie eingangs definiert sind,
einer der Reste $E_1$ oder $G_1$ die für E oder G eingangs erwähnten Bedeutungen aufweist und
der andere der Reste $E_1$ oder $G_1$ ebenfalls die für E oder G eingangs erwähnten Bedeutungen besitzt, wobei jedoch eine zu einem Stickstoffatom benachbarte Methylengruppe durch eine Carbonylgruppe ersetzt sein muß.

Die Reduktion wird vorzugsweise in einem geeigneten Lösungsmittel wie Methanol, Ethanol, Diethylether oder Tetrahydrofuran in Gegenwart eines Metallhydrids wie Natriumborhydrid, Lithiumaluminiumhydrid, Diboran, Boran/Dimethylsulfid oder Natriumcyanborhydrid, vorzugsweise jedoch mit Natriumborhydrid in Methanol oder Ethanol, zwischen 0 und 40 ° C, vorzugsweise jedoch bei Raumtemperatur, durchgeführt.

Bei der Reduktion mit einem komplexen Metallhydrid wie Lithiumaluminiumhydrid, Diboran oder Boran/Dimethylsulfid, kann eine im Rest A vorhandene Carbonylfunktion zu einer Methylengruppe mitreduziert werden.

e) Zur Herstellung von Verbindungen der allgemeinen Formel I, in der die -G-L-Gruppe eine gegebenenfalls durch eine Alkylgruppe substituierte Ethylengruppe darstellt:
Umsetzung einer Verbindung der allgemeinen Formel

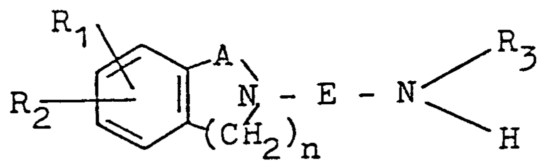

(IX)

in der

$R_1$ bis $R_3$, A, E und n wie eingangs definiert sind, mit einer Vinylverbindung der allgemeinen Formel

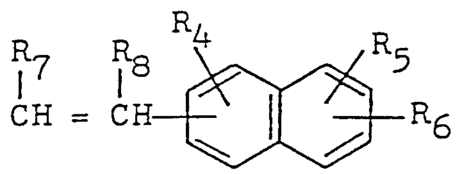

(X)

in der

$R_4$ bis $R_6$ wie eingangs definiert sind,

einer der Reste $R_7$ oder $R_8$ ein Wasserstoffatom und der andere der Reste $R_7$ oder $R_8$ eine Alkylgruppe mit 1 bis 3 Kohlenstoffatomen bedeuten.

Die Umsetzung wird vorzugsweise in einem Lösungsmittel wie Methanol, Ethanol, Dioxan oder Tetrahydrofuran bei Temperaturen zwischen 0 und 50 ° C, vorzugsweise durch Stehen bei Raumtemperatur durchgeführt.

f) Zur Herstellung von 2-[N-Methyl-N-((naphth-1-oxy)-eth-2-yl)-amino-prop-3-yl]-5,6-dimethoxy-phthalimidin, 2-[N-Methyl-N-((naphth-2-yl)-methyl)-amino-prop-3-yl]-5,6-dimethoxyphthalimidin, 2-[N-Methyl-N-((naphth-2-yl-oxy)-but-4-yl)-amino-prop-3-yl]-5,6-dimethoxy-phthalimidin, 2-[N-Methyl-N-((naphth-1-yl)-eth-2-yl)-amino-prop-3-yl]-5,6-dimethoxyphthalimidin, 2-[N-Methyl-N-((5-methyl-6-methoxy-naphth-2-yl)-eth-2-yl)-amino-prop-3-yl]-5,6-dimethoxy-phthalimidin und 2-[N-Methyl-N-((2-methyl-naphth-1-yl)-methyl)-amino-prop-3-yl]-5,6-dimethoxy-phthalimidin:

Reduktion eines entsprechenden Phthalimids durch 3-stündiges Erhitzen in Zink/Eisessig unter Rückfluß.

Bei den vorstehend beschriebenen Umsetzungen a) bis e) können gegebenenfalls vorhandene reaktive Gruppen wie Amino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Benzyl- oder Tetrahydropyranylgruppe und

als Schutzrest für eine Imino- oder Aminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 ° C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und einem Wasserstoffdruck von 1 bis 7 bar, vorzugsweise jedoch von 3 bis 5 bar.

Die erhaltenen Verbindungen der allgemeinen Formel I lassen sich, falls diese ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure oder D- oder L-Camphersäure.

Die erhaltenen Verbindungen der allgemeinen Formel I lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure,

Weinsäure, Bernsteinsäure, Maleinsäure oder Fumarsäure in Betracht.

Die als Ausgangsstoffe verwendeten Verbindungen der allgemeinen Formeln II bis XI sind teilweise literaturbekannt bzw. man erhält sie nach an sich bekannten Verfahren.

So erhält man beispielsweise ein in 3-Stellung unsubstituiertes Benzazepin der allgemeinen Formel IV durch Cyclisierung einer entsprechenden Verbindung, z.B. durch Cyclisierung einer Verbindung der allgemeinen Formel

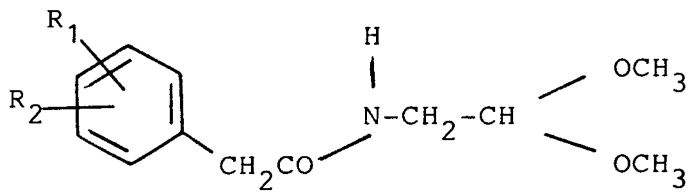

(XII)

oder auch der allgemeinen Formel

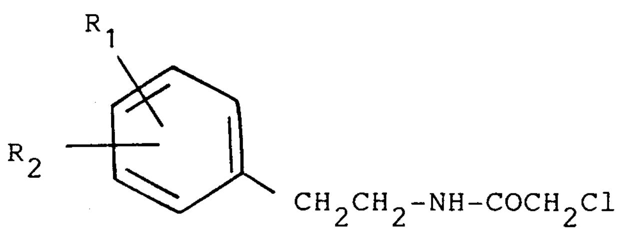

(XIII)

gegebenenfalls anschließender katalytischer Hydrierung und/oder Reduktion der Carbonylgruppe beispielsweise mit Natriumborhydrid/Eisessig (siehe EP-A1 0.007.070, EP-A1 0.065.229 und EP-A1 0.109.639) und/oder Oxidation, z.B. mit Selendioxid oder durch Umsetzung eines entsprechend substituierten Phenyläthylamins mit Chloressigsäurechlorid und anschließender Cyclisierung (siehe Tetrahedron Letters 21, 1393 (1980)).

Ein Isochinolin-1-on der allgemeinen Formel IV erhält man durch Umsetzung eines entsprechend substituierten Phenyläthylamines mit einem Chlorameisensäureester und anschließender Cyclisierung (siehe Helv. Chim. Acta 47, 2692 (1964)) bzw. durch Überführung eines entsprechend substituierten β-Phenylpropionsäure in das Isocyanat und anschließender Cyclisierung (siehe Chem. Pharm. Bull. Jap. 24, 2976 (1979)).

Ein Phthalimidin der allgemeinen Formel II oder ein 1H-Phthalimidin der allgemeinen Formel IV erhält man durch Reduktion eines entsprechenden Phthalimids, z. B. mit Zinkstaub/Eisessig.

Eine Ausgangsverbindung der allgemeinen Formeln II, VI und IX erhält man durch Alkylierung einer Verbindung der allgemeinen Formel IV mit einem entsprechenden Halogenalkan und gegebenenfalls anschließende Umsetzung mit einem entsprechenden Amin.

Eine Ausgangsverbindung der allgemeinen Formel III erhält man durch Umsetzung einer entsprechenden Hydroxyverbindung mit einem entsprechenden Sulfonsäurehalogenid oder einem entsprechenden Halogenierungsmittel und gegebenenfalls anschließende Umsetzung mit einem entsprechenden Amin, wobei eine so erhaltene Aminoverbindung der allgemeinen Formel VII durch Umsetzung mit einem entsprechenden Dihalogenalkan in eine Verbindung der allgemeinen Formel V übergeführt werden kann.

Eine Carbonylverbindung der allgemeinen Formel VI erhält man durch Umsetzung einer Verbindung der allgemeinen Formel IV mit einem entsprechenden Halogen-alkanal und eine Carbonylverbindung der allgemeinen Formel VII durch Oxidation einer entsprechenden Hydroxyverbindung.

Eine Verbindung der allgemeinen Formel VIII erhält man durch Umsetzung eines entsprechenden Amins mit einer entsprechenden Carbonsäure und eine Vinylverbindung der allgemeinen Formel X durch Dehydratisierung einer entsprechenden Hydroxyverbindung.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen zentralen Nebenwirkungen eine besonders lang anhaltende herzfrequenzsenkende Wirkung sowie eine Herabsetzung des $0_2$-Bedarfs des Herzens.

Beispielsweise wurden die Verbindungen

A = 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on,
B = 3-[N-Methyl-N-((6-methoxy-5-methyl-naphth-2-yl-)-eth-2-yl)-amino-prop-3-yl]-7,8-dimethoxy-1,3,4,5-tetrahydro-2H-3-benzazepin-2-on,
C = 3-[N-Methyl-N-((naphth-2-yl)-eth-2-yl)-amino-prop-3-yl]-7,8-dimethoxy-1,3,4,5-tetrahydro-2H-3-benzazepin-2-on,
D = 2-[N-Methyl-N-((naphth-2-yl)-eth-2-yl)-amino-prop-3-yl]-6,7-methylendioxy-1,2,3,4-tetrahydro-isochinolin-1-on-hydrochlorid und
E = 2-[N-Methyl-N-((5-methyl-6-methoxy-naphth-2-yl)-eth-2-yl)-amino-prop-3-yl]-6,7-dimethoxy-1,2,3,4-tetrahydroisochinolin-1-on-hydrochlorid

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung, gemessen 20 Minuten nach Substanzapplikation [S/min] |
|---|---|---|
| A | 5,0 | - 184 |
| B | 5,0 | - 177 |
| C | 5,0 | - 170 |
| D | 5,0 | - 163 |
| E | 5,0 | - 91 |

Die erfindungsgemäß hergestellten Verbindungen weisen in therapeutischen Dosen keinerlei toxische Nebenwirkungen auf. So konnten beipielsweise bei einer intravenösen Applikation der Substanz A bis E auch in einer hohen Dosis von 20 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,01 bis 0,2 mg/kg Körpergewicht, vorzugsweise 0,03 bis 0,15 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Sauren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/ Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Beispiel 1

2-[N-Methyl-N-((2-methyl-naphth-1-yl)-eth-2-yl)-amino-prop-3-yl]-6,7-dimethoxy-1,2,3,4-tetrahydro-isochinolin-1-on-hydrochlorid

Ein Gemisch von 1,13 g (4 mMol) 2-(3-Chlorpropyl)-6,7-dimethoxy-1,2,3,4-tetrahydro-isochinolin-1-on, 1,04 g (4,4 mMol) N-Methyl-2-(2-methyl-naphth-1-yl)-ethylamin-hydrochloridund 1,5 ml N-Ethyl-diisopropylamin wird 2 Stunden unter Rückfluß erhitzt. Das überschüssige N-Ethyl-diisopropylamin wird im Vakuum abgedampft und der verbleibene Rückstand in einem Gemisch von Methylenchlorid und 2 molarer Natronlauge gelöst. Die organische Phase wird abgetrennt, mit Wasser gewaschen, über Magnesiumsulfat getrocknet, im Vakuum eingedampft und über eine 150 g Kieselgelsäule (0,062-0,2 mm) mit Methylenchlorid und anschließend steigenden Anteilen von Ethanol (bis 5 %) gereinigt. Aus einer Lösung in Aceton wird mit etherischer Salzsäure das Hydrochlorid gefällt, welches aus Aceton umkristallisiert wird.
Ausbeute: 0,67 g (34,7 % der Theorie),
Schmelzpunkt: 130-132 °C

| | | | | |
|---|---|---|---|---|
| Ber.: | C 69,62 | H 7,20 | N 5,80 | Cl 7,34 |
| Gef.: | 69,53 | 7,46 | 5,84 | 7,64 |

Beispiel 2

2-[N-Methyl-N-((naphth-2-yl)-prop-2-yl)-amino-prop-3-yl]-6,7-dimethoxy-1,2,3,4-tetrahydro-isochinolin-1-on-hydrochlorid

0,83 g (4 mMol) 6,7-Dimethoxy-1,2,3,4-tetrahydro-isochinolin-1-on wird in 15 ml Dimethylformamid gelöst und unter Rühren mit 0,49 g (4,4 mMol) Kalium-tert.butylat versetzt. Das Kaliumsalz fällt nach exothermer Reaktion (bis ca. 40 °C) nach ca. 30 Minuten aus. Es wird auf 0 °C abgekühlt und 1,4 g (4,4 mMol) 3-[N-Methyl-(naphth-2-yl)-prop-2-yl-amino]-propylbromid zugefügt. Nach 4 Stunden bei dieser Temperatur wird auf Eiswasser gegossen und mit Essigester ausgeschüttelt. Die organische Phase wird mit Wasser gewaschen, über Magnesiumsulfat getrocknet, im Vakuum eingedampft und über eine Kieselgelsäule (0,063-0,2 mm) mit Methylenchlorid und anschließend steigenden Anteilen Ethanol (bis 5 %) gereinigt. Aus einer Lösung in Aceton wird mit etherischer Salzsäure das Hydrochlorid gefällt, welches aus Aceton umkristallisiert wird.
Ausbeute: 0,65 g (33,7 % der Theorie),
Schmelzpunkt: 133-135 °C

| | | | | |
|---|---|---|---|---|
| Ber.: | C 69,62 | H 7,30 | N 5,80 | Cl 7,34 |
| Gef.: | 69,35 | 7,41 | 5,89 | 7,52 |

Beispiel 3

2-[N-Methyl-N-((5-methyl-6-methoxy-naphth-2-yl)-eth-2-yl)-amino-prop-3-yl]-6,7-dimethoxy-1,2,3,4-tetrahydro-isochinolin-1-on-hydrochlorid

0,83 g (4 mMol) 6,7-Dimethoxy-1,2,3,4-tetrahydro-isochinolin-1-on wird in 15 ml Dimethylformamid gelöst und unter Rühren mit 0,49 g (4,4 mMol) Kalium-tert.butylat versetzt. Das Kaliumsalz fällt nach exothermer Reaktion (bis ca. 40 °C) nach ca. 30 Minuten aus. Es wird auf 0 °C abgekühlt und 1,9 g (4,4 mMol) Benzolsulfonsäure-3-[N-methyl-((5-methyl-6-methoxy-naphth-2-yl)-eth-2-yl)-propyl-amino]-propylester zugefügt. Nach 4 Stunden bei dieser Temperatur wird auf Eiswasser gegossen und mit Essigester ausgeschüttelt. Die organische Phase wird mit Wasser gewaschen, über Magnesiumsulfat getrocknet, im Vakuum eingedampft und über eine Kieselgelsäule (0,063-0,2 mm) mit Methylenchlorid und anschließend steigenden Anteilen Ethanol (bis 5 %) gereinigt. Aus einer Lösung in Aceton wird mit etherischer Salzsäure das Hydrochlorid gefällt, welches aus Aceton umkristallisiert wird.
Ausbeute: 0,45 g (21,9 % der Theorie),
Schmelzpunkt: 187-189 °C

| | | | | |
|---|---|---|---|---|
| Ber.: | C 67,89 | H 7,27 | N 5,46 | Cl 6,91 |
| Gef.: | 67,70 | 7,28 | 5,44 | 6,88 |

Beispiel 4

3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

Ein Gemisch von 0,66 g (2,5 mMol) 2-(2-Bromethyl)-6-methoxy-naphthalin und 1,46 g (5 mMol) 3-(N-Methylamino-prop-3-yl)-7,8-dimethoxy-1,3,4,5-tetrahydro-2H-3-benzazepin-2-on wird 90 Minuten auf 100°C erhitzt. Das abgekühlte Rohprodukt wird über 70 g Aluminiumoxid (neutral, Aktivität II-III) mit Methylenchlorid und anschließend mit steigenden Anteilen von Ethanol (bis 0,25 %) gereinigt.
Ausbeute: 0,45 g (37,8 % der Theorie),
Schmelzpunkt: 93-96°C

| | | | |
|---|---|---|---|
| Ber.: | C 73,08 | H 7,61 | N 5,88 |
| Gef.: | 73,20 | 7,58 | 5,71 |

Beispiel 5

2-[N-Methyl-N-((naphth-2-oxy)-but-4-yl)-amino-prop-3-yl]-6,7-dimethoxy-1,2,3,4-tetrahydro-isochinolin-1-on-hydrochlorid

1,05 g (4 mMol) 2-(2-Formyl-ethyl)-6,7-dimethoxy-1,2,3,4-tetrahydro-isochinolin-1-on, 0,92 g (4 mMol) N-Methyl-4-(naphth-2-oxy)-butylamin werden in 50 ml Ethanol in Gegenwart von 0,2 g 10%igem Palladium auf Aktivkohle bei 70°C und 5 bar bis zur Aufnahme der berechneten Menge mit Wasserstoff hydriert. Der Katalysator wird abfiltriert und das Filtrat im Vakuum zur Trockene eingedampft. Aus einer Lösung in Aceton wird mit etherischer Salzsäure das Hydrochlorid gefällt, welches aus Aceton/Ether umkristallisiert wird.
Ausbeute: 0,6 g (60 % der Theorie),
Schmelzpunkt: 135-136°C

| | | | | |
|---|---|---|---|---|
| Ber.: | C 67,89 | H 7,27 | N 5,46 | Cl 6,91 |
| Gef.: | 67,81 | 7,22 | 5,42 | 6,79 |

Die Reduktion kann auch mit Natriumborhydrid in Ethanol bei Raumtemperatur oder Siedetemperatur durchgeführt werden.

Beispiel 6

2-[N-Methyl-N-((2-methyl-naphth-1-yl)-methyl)-amino-prop-3-yl]-6,7-dimethoxy-1,2,3,4-tetrahydro-isochinolin-1-on-hydrochlorid

a) 2-[N-Methyl-N-(2-methyl-naphth-1-yl)-amido-prop-3-yl]-6,7-dimethoxy-1,2,3,4-tetrahydro-isochinolin-1-on

Zu einer Lösung von 0,82 g (4,4 mMol) 2-Methyl-1-naphthoesäure in 20 ml Dimethylformamid werden 0,71 g (4,4 mMol) N,N'-Carbonyldiimidazol gegeben. Unter Kohlensäureentwicklung wird während ca. 30 Minuten das Imidazolid der Säure gebildet. Es werden 1,06 g (4 mMol) 2-[N-Methyl-amino-prop-3-yl]-6,7-dimethoxy-1,2,3,4-tetrahydro-isochinolin-1-on zugefügt und 2 Stunden bei Raumtemperatur gerührt. Das Lösungsmittel wird im Vakuum abgedampft, der verbliebene Rückstand in einem Gemisch von 2 molarer Natronlauge und Methylenchlorid gelöst. Die organische Phase wird mit Wasser gewaschen, über Magnesiumsulfat getrocknet und im Vakuum eingedampft.
Rohausbeute: 1,3 g (67,3 % der Theorie)

b) 2-[N-Methyl-N-((2-methyl-naphth-1-yl)-methyl)-amino-prop-3-yl]-6,7-dimethoxy-1,2,3,4-tetrahydro-isochinolin-1-on-hydrochlorid

Zu einer Lösung von 1,3 g (3 mMol) 2-[N-Methyl-N-(2-methyl-naphth-1-yl)-amido-prop-3-yl]-6,7-dimethoxy-1,2,3,4-tetrahydro-isochinolin-1-on in 50 ml Tetrahydrofuran werden 4,5 ml 1 molarer Boran-Tetrahydrofuran-Komplex in Tetrahydrofuran zugegeben und unter Rühren bei Raumtemperatur 0,55 ml (4,5 mMol) Bortrifluorid-diethyletherat-Komplex zugetropft. Nach 3 Stunden Reaktionszeit wird tropfenweise mit 5 ml 6 molarer Salzsäure versetzt, 0,5 Stunden am Rückfluß erhitzt und danach das Lösungsmittel im Vakuum abgedampft. Der verbliebene wässrige Teil wird mit 2 molarer Natronlauge alkalisch gemacht und mit Methylenchlorid ausgeschüttelt. Die organische Phase wird mit Wasser gewaschen, über Natriumsulfat getrocknet, im Vakuum eingedampft und über eine Kieselgelsäule (0,063-0,2 mm) mit Methylenchlorid und anschließend steigenden Anteilen von Ethanol (bis 5 %) gereinigt. Aus einer Lösung in Aceton wird mit etherischer Salzsäure das Hydrochlorid gefällt, welches aus Aceton umkristallisiert wird.
Ausbeute: 0,21 g (10,2 % der Theorie),
Schmelzpunkt: 187-189 ° C

| | | | | |
|---|---|---|---|---|
| Ber.: | C 69,15 | H 7,09 | N 5,97 | Cl 7,56 |
| Gef.: | 69,00 | 7,20 | 6,12 | 7,49 |

Beispiel 7

2-[N-Methyl-N-((6-methoxy-naphth-2-yl)-eth-2-yl)-amino-prop-3-yl]-6,7-methylendioxy-1,2,3,4-tetrahydro-isochinolin-1-on-hydrochlorid

1,4 g (5 mMol) 2-[N-Methyl-amino-prop-3-yl]-6,7-methylendioxy-1,2,3,4-tetrahydro-isochinolin-1-on werden in 20 ml Methanol gelöst und 1 g (5,5 mMol) 6-Methoxy-2-vinyl-naphthlin zugefügt. Nach dem Stehen über Nacht bei Raumtemperatur wird das Lösungsmittel im Vakuum abgedampft und über eine Kieselgelsäule (0,063-0,2 mm) mit Methylenchlorid und anschließend ansteigenden Anteilen von Ethanol (bis 5 %) gereinigt. Aus einer Lösung in Aceton wird mit etherischer Salzsäure das Hydrochlorid gefällt, welches aus Aceton/Ether umkristallisiert wird.
Ausbeute: 0,28 g (11,6 % der Theorie),
Schmelzpunkt: 205-207 ° C

| | | | | |
|---|---|---|---|---|
| Ber.: | C 67,14 | H 6,47 | N 5,80 | Cl 7,34 |
| Gef.: | 66,90 | 6,58 | 5,72 | 7,38 |

Beispiel 8

2-[N-Methyl-N-((naphth-1-oxy)-eth-2-yl)-amino-prop-3-yl]-5,6-dimethoxy-phthalimidin

850 mg (1,9 mMol) 2-[N-Methyl-N-((naphth-1-oxy)-eth-2-yl)-amino-prop-3-yl]-5,6-dimethoxy-phthalimid werden in 10 ml Eisessig gelöst. Nach der Zugabe von 900 mg (13,8 mMol) Zink-Pulver wird unter Rühren 3 Stunden zum Rückfluß erhitzt. Das Lösungsmittel wird im Vakuum abdestilliert und der Rückstand in Natronlauge (w = 15 %) und Essigsäureethylester verteilt. Die organische Phase wird mit Natriumsulfat getrocknet, eingeengt und über eine Kieselgel-Flash-Säule mit Methylenchlorid/Methanol als Elutionsmittel gereinigt, wobei man 460 mg (56 % der Theorie) eines gelben Öls erhält. Man löst in absolutem Methanol und fällt mit etherischer Salzsäure das Hydrochlorid.
Ausbeute: 380 mg (42 % der Theorie),
$R_f$-Wert der Base: 0,35 (Kieselgel, Methylenchlorid/Methanol = 9/1).

Beispiel 9

3-[N-Methyl-N-((naphth-2-yl)-eth-2-yl)-amino-prop-3-yl]-6,7-dimethoxy-1,2,3,4-tetrahydro-isochinolin-1-on-hydrochlorid

Hergestellt aus 3-(3-Chlorpropyl)-6,7-dimethoxy-1,2,3,4-tetrahydro-isochinolin-1-on und N-Methyl-2-(naphth-2-yl)-ethylamin analog Beispiel 1.
Ausbeute: 50,8 % der Theorie,
Schmelzpunkt: 163-165 ° C

| | | | | |
|---|---|---|---|---|
| Ber.: | C 69,14 | H 7,09 | N 5,97 | Cl 7,56 |
| Gef.: | 68,91 | 6,95 | 6,18 | 7,35 |

Beispiel 10

2-[N-Methyl-N-((2-methyl-naphth-1-yl)-eth-2-yl)-amino-prop-3-yl]-6,7-methylendioxy-1,2,3,4-tetrahydro-isochinolin-1-on-hydrochlorid

Hergestellt aus 2-(3-Chlorpropyl)-6,7-methylendioxy-1,2,3,4-tetrahydro-isochinolin-1-on-hydrochlorid und N-Methyl-2-(2-methyl-naphth-1-yl)-ethylamin analog Beispiel 1.
Ausbeute: 36,6 % der Theorie,
Schmelzpunkt: 168-170 ° C

| | | | | |
|---|---|---|---|---|
| Ber.: | C 69,44 | H 6,69 | N 5,99 | Cl 7,59 |
| Gef.: | 69,34 | 6,79 | 6,00 | 7,78 |

Beispiel 11

2-[N-Methyl-N-(2-(naphth-2-yl)-prop-1-yl)-amino-prop-3-yl]-6,7-dimethyl-1,2,3,4-tetrahydro-isochinolin-1-on

Hergestellt aus 2-(3-Chlorpropyl)-6,7-dimethyl-1,2,3,4-tetrahydro-isochinolin-1-on und N-Methyl-2-(naphth-2-yl)-propylamin analog Beispiel 1.
Ausbeute: 45,9 % der Theorie,
Schmelzpunkt: 114-116 ° C

| | | | |
|---|---|---|---|
| Ber.: | C 81,12 | H 8,27 | N 6,76 |
| Gef.: | 80,92 | 8,06 | 6,70 |

Beispiel 12

2-[N-Methyl-N-((naphth-2-yl)-eth-2-yl)-amino-prop-3-yl]-6,7-methylendioxy-1,2,3,4-tetrahydro-isochinolin-1-on-hydrochlorid

Hergestellt aus 2-(3-Chlorpropyl)-6,7-methylendioxy-1,2,3,4-tetrahydro-isochinolin-1-on und N-Methyl-2-(naphth-2-yl)-ethylamin analog Beispiel 1.
Ausbeute: 40,7 % der Theorie,
Schmelzpunkt: 188-190 ° C

| | | | | |
|---|---|---|---|---|
| Ber.: | C 69,09 | H 6,24 | N 6,19 | Cl 7,84 |
| Gef.: | 69,08 | 6,60 | 6,08 | 8,08 |

Beispiel 13

2-[N-Methyl-N-((naphth-2-yl)-prop-3-yl)-amino-prop-3-yl]-6,7-dimethoxy-1,2,3,4-tetrahydro-isochinolin-1-on-hydrochlorid

Hergestellt aus 2-(3-Chlorpropyl)-6,7-dimethoxy-1,2,3,4-tetrahydro-isochinolin-1-on und N-Methyl-3-(naphth-2-yl)-propylamin analog Beispiel 1.
Ausbeute: 26 % der Theorie,
Schmelzpunkt: 76-78 ° C

| | | | | |
|---|---|---|---|---|
| Ber.: | C 69,62 | H 7,30 | N 5,80 | Cl 7,34 |
| Gef.: | 69,50 | 7,18 | 5,60 | 7,31 |

Beispiel 14

2-[N-Methyl-N-((naphth-2-yl)-prop-3-yl)-amino-prop-3-yl]-6,7-methylendioxy-1,2,3,4-tetrahydro-isochinolin-1-on-hydrochlorid

Hergestellt aus 2-(3-Chlorpropyl)-6,7-methylendioxy-1,2,3,4-tetrahydro-isochinolin-1-on und N-Methyl-3-(naphth-2-yl)-propylamin analog Beispiel 1.
Ausbeute: 17,8 % der Theorie,
Schmelzpunkt: 74-78 ° C

| | | | | |
|---|---|---|---|---|
| Ber.: | C 69,44 | H 6,69 | N 5,99 | Cl 7,59 |
| Gef.: | 69,30 | 6,69 | 5,98 | 7,49 |

Beispiel 15

2-[N-Methyl-N-((naphth-2-yl)-prop-3-yl)-amino-prop-3-yl]-6,7-dimethyl-1,2,3,4-tetrahydro-isochinolin-1-on-hydrochlorid

Hergestellt aus 2-(3-Chlorpropyl)-6,7-dimethyl-1,2,3,4-tetrahydro-isochinolin-1-on und N-Methyl-3-(naphth-2-yl)-propylamin analog Beispiel 1.
Ausbeute: 26,1 % der Theorie,
Schmelzpunkt: 148-149 ° C

| | | | | |
|---|---|---|---|---|
| Ber.: | C 74,57 | H 7,82 | N 6,21 | Cl 7,86 |
| Gef.: | 74,37 | 7,64 | 6,21 | 7,91 |

Beispiel 16

2-[N-Methyl-N-((5-methyl-6-methoxy-naphth-2-yl)-eth-2-yl)-amino-prop-3-yl]-6,7-methylendioxy-1,2,3,4-tetrahydro-isochinolin-1-on-hydrochlorid

Hergestellt aus 2-(3-Chlorpropyl)-6,7-methylendioxy-1,2,3,4-tetrahydro-isochinolin-1-on und N-Methyl-2-(5-methyl-6-methoxy-naphth-2-yl)-ethylamin analog Beispiel 1.
Ausbeute: 27 % der Theorie,
Schmelzpunkt: 234-236 ° C

| | | | | |
|---|---|---|---|---|
| Ber.: | C 67,66 | H 6,69 | N 5,63 | Cl 7,13 |
| Gef.: | 67,70 | 6,59 | 5,60 | 7,22 |

Beispiel 17

2-[N-Methyl-N-((naphth-2-oxy)-but-4-yl)-amino-prop-3-yl]-6,7-methylendioxy-1,2,3,4-tetrahydro isochinolin-1-on-hydrochlorid

Hergestellt aus 2-(3-Chlorpropyl)-6,7-methylendioxy-1,2,3,4-tetrahydro-isochinolin-1-on und N-Methyl-4-(naphth-2-oxy)-butylamin analog Beispiel 1.
Ausbeute: 31 % der Theorie,
Schmelzpunkt: 157-160 ° C

| | | | | |
|---|---|---|---|---|
| Ber.: | C 67,66 | H 6,69 | N 5,63 | Cl 7,13 |
| Gef.: | 67,60 | 6,67 | 5,63 | 7,33 |

Beispiel 18

2-[N-Methyl-N-((6-methoxy-naphth-2-yl)-eth-2-yl)-amino-prop-3-yl]-6,7-dimethyl-1,2,3,4-tetrahydro-isochinolin-1-on-hydrochlorid

Hergestellt aus 2-(3-Chlorpropyl)-6,7-dimethyl-1,2,3,4-tetrahydro-isochinolin-1-on und N-Methyl-2-(6-methoxy-naphth-2-yl)-ethylamin analog Beispiel 1.
Ausbeute: 28,4 % der Theorie,
Schmelzpunkt: 212-214 ° C

| | | | | |
|---|---|---|---|---|
| Ber.: | C 72,01 | H 7,55 | N 6,00 | Cl 7,59 |
| Gef.: | 71,80 | 7,40 | 5,96 | 7,64 |

Beispiel 19

2-[N-Methyl-N-((2-methyl-naphth-1-yl)-methyl)-amino-prop-3-yl]-6,7-dimethyl-1,2,3,4-tetrahydro-isochinolin-1-on-hydrochlorid

Hergestellt aus 2-(3-Chlorpropyl)-6,7-dimethyl-1,2,3,4-tetrahydro-isochinolin-1-on und N-Methyl-(2-methyl-naphth-1-yl)-methylamin analog Beispiel 1.
Ausbeute: 39,4 % der Theorie,
Schmelzpunkt: 205-206 ° C

| | | | | |
|---|---|---|---|---|
| Ber.: | C 74,21 | H 7,61 | N 6,41 | Cl 8,11 |
| Gef.: | 73,98 | 7,47 | 6,26 | 8,34 |

Beispiel 20

3-[N-Methyl-N-((6-methoxy-5-methyl-naphth-2-yl)-eth-2-yl)-amino-prop-3-yl]-7,8-dimethoxy-1,3,4,5-tetrahydro-2H-3-benzazepin-2-on

Hergestellt aus 3-[(N-Methylamino)-prop-3-yl]-7,8-dimethoxy-1,3,4,5-tetrahydro-2H-3-benzazepin-2-on und 2-(2-Bromethyl)-6-methoxy-5-methyl-naphthalin analog Beispiel 4.
Ausbeute: 44,7 % der Theorie,
Schmelzpunkt: 88-92 ° C

| | | | |
|---|---|---|---|
| Ber.: | C 73,44 | H 7,81 | N 5,71 |
| Gef.: | 73,32 | 7,62 | 5,58 |

Beispiel 21

3-[N-Methyl-N-((2-methyl-naphth-1-yl)-methyl)-amino-prop-3-yl]-7,8-dimethoxy-1,3,4,5-tetrahydro-2H-3-benzazepin-2-on

Hergestellt aus 3-[(N-Methylamino)-prop-3-yl]-7,8-dimethoxy-1,3,4,5-tetrahydro-2H-3-benzazepin-2-on und 1-Chlormethyl-2-methyl-naphthalin analog Beispiel 4.
Ausbeute: 80,4 % der Theorie,
Schmelzpunkt: 114-116 ° C

| | | | |
|---|---|---|---|
| Ber.: | C 75,31 | H 7,67 | N 6,27 |
| Gef.: | 75,15 | 7,65 | 6,06 |

Beispiel 22

3-[N-Methyl-N-((naphth-1-yl)-eth-2-yl)-amino-prop-3-yl]-7,8-dimethoxy-1,3,4,5-tetrahydro-2H-3-benzazepin-2-on-dihydrochlorid

Hergestellt aus 3-(3-Chlorpropyl)-7,8-dimethoxy-1,3,4,5-tetrahydro-2H-3-benzazepin-2-on und 2-(N-Methylamino)-(1-naphthyl)-ethan analog Beispiel 1.
Ausbeute: 11,3 % der Theorie,
Schmelzpunkt: 151-155 ° C

| | | | | |
|---|---|---|---|---|
| Ber.: | C 66,79 | H 7,20 | N 5,56 | Cl 14,08 |
| Gef.: | 66,62 | 7,12 | 5,29 | 13,80 |

Beispiel 23

3-[N-Methyl-N-(2-(naphth-1-yl)-prop-1-yl)-amino-prop-3-yl]-7,8-dimethoxy-1,3,4,5-tetrahydro-2H-3-benzazepin-2-on-hydrochlorid

Hergestellt aus 3-(3-Chlorpropyl)-7,8-dimethoxy-1,3,4,5-tetrahydro-2H-3-benzazepin-2-on und 1-(N-Methylamino)-2-(1-naphthyl)-propan analog Beispiel 1.
Ausbeute: 21,7 % der Theorie,
Schmelzpunkt: 138 ° C (Sinterung ab 108 ° C)

| | | | | |
|---|---|---|---|---|
| Ber.: | C 70,70 | H 7,50 | N 5,64 | Cl 7,13 |
| Gef.: | 70,45 | 7,42 | 5,39 | 7,38 |

Beispiel 24

3-[N-Methyl-N-((naphth-2-oxy)-but-4-yl)-amino-prop-3-yl]-7,8-dimethoxy-1,3,4,5-tetrahydro-2H-3-benzazepin-2-on-hydrochlorid

Hergestellt aus 3-(3-Chlorpropyl)-7,8-dimethoxy-1,3,4,5-tetrahydro-2H-3-benzazepin-2-on und 1-(N-Methylamino)-4-(2-naphthoxy)-butan analog Beispiel 1.
Ausbeute: 25,5 % der Theorie,
Schmelzpunkt: 210-212 ° C

| | | | | |
|---|---|---|---|---|
| Ber.: | C 68,36 | H 7,46 | N 5,31 | Cl 6,73 |
| Gef.: | 68,39 | 7,37 | 5,30 | 6,59 |

Beispiel 25

3-[N-Methyl-N-((2-methyl-naphth-1-yl)-eth-2-yl)-amino-prop-3-yl]-7,8-dimethoxy-1,3,4,5-tetrahydro-2H-3-benzazepin-2-on-hydrochlorid

Hergestellt aus 3-(3-Chlorpropyl)-7,8-dimethoxy-1,3,4,5-tetrahydro-2H-3-benzazepin-2-on und 1-(N-Methylamino)-2-(2-methyl-naphth-1-yl)-ethan analog Beispiel 1.
Ausbeute: 34,8 % der Theorie,
Schmelzpunkt: 217-219 ° C

| | | | | |
|---|---|---|---|---|
| Ber.: | C 70,07 | H 7,50 | N 5,64 | Cl 7,13 |
| Gef.: | 69,91 | 7,45 | 5,75 | 7,29 |

Beispiel 26

3-[N-Methyl-N-((naphth-1-yl)-eth-2-yl)-amino-prop-3-yl]-7,8-dimethoxy-1,3,4,5-tetrahydro-2H-3-benzazepin-1,2-dion-hydrochlorid

Hergestellt aus 3-(3-Chlorpropyl)-7,8-dimethoxy-1,3,4,5-tetrahydro-2H-3-benzazepin-1,2-dion und 1-(N-Methylamino)-2-(1-naphthyl)-ethan analog Beispiel 1.
Ausbeute: 13,9 % der Theorie,
Schmelzpunkt: 244-246 ° C

| | | | | |
|---|---|---|---|---|
| Ber.: | C 67,65 | H 6,69 | N 5,64 | Cl 7,13 |
| Gef.: | 67,55 | 6,49 | 5,81 | 7,18 |

Beispiel 27

2-[N-Methyl-N-((naphth-2-yl)-methyl)-amino-prop-3-yl]-5,6-dimethoxy-phthalimidin

Hergestellt aus 2-[N-Methyl-N-(naphth-2-yl)-methyl-amino-prop-3-yl]-5,6-dimethoxy-phthalimid und Zink/Eisessig analog Beispiel 8.
Ausbeute: 43 % der Theorie,

| | | | | |
|---|---|---|---|---|
| Ber.: | C 62,89 | H 6,33 | N 5,87 | Cl 14,85 |
| Gef.: | 63,00 | 6,54 | 6,02 | 14,68 |

$R_f$-Wert: 0,45 (Kieselgel, Methylenchlorid/Methanol = 9:1)

Beispiel 28

2-[N-Methyl-N-((naphth-2-yl-oxy)-but-4-yl)-amino-prop-3-yl)-5,6-dimethoxy-phthalimidin

Hergestellt aus 2-[N-Methyl-N-((naphth-2-yl-oxy)-but-4-yl)-amino-prop-3-yl]-5,6-dimethoxy-phthalimid und Zink/Eisessig analog Beispiel 8.
Ausbeute: 12 % der Theorie,

| | | | |
|---|---|---|---|
| Ber.: | C 63,04 | H 6,42 | Cl 13,29 |
| Gef.: | 62,98 | 6,34 | 13,73 |

$R_f$-Wert: 0,29 (Kieselgel, Methylenchlorid/Methanol = 9/1)

Beispiel 29

2-[N-Methyl-N-((naphth-1-yl)-eth-2-yl)-amino-prop-3-yl]-5,6-dimethoxy-phthalimidin

Hergestellt aus 2-[N-Methyl-N-((naphth-1-yl)-eth-2-yl)-amino-prop-3-yl]-5,6-dimethoxy-phthalimid und Zink/Eisessig analog Beispiel 8.
Ausbeute: 58 % der Theorie,

| | | | | |
|---|---|---|---|---|
| Ber.: | C 68,63 | H 6,87 | N 6,16 | Cl 7,79 |
| Gef.: | 68,72 | 7,04 | 6,10 | 7,93 |

$R_f$-Wert: 0,32 (Kieselgel, Methylenchlorid/Methanol = 9/1)

Beispiel 30

2-[N-Methyl-N-((5-methyl-6-methoxy-naphth-2-yl)-eth-2-yl)-amino-prop-3-yl]-5,6-dimethoxy-phthalimidin

Hergestellt aus 2-[N-Methyl-N-((5-methyl-6-methoxy-naphth-2-yl)-eth-2-yl)-amino-prop-3-yl]-5,6-dimethoxy-phthalimid und Zink/Eisessig analog Beispiel 8.
Ausbeute: 74 % der Theorie,

| | | | | |
|---|---|---|---|---|
| Ber.: | C 67,39 | H 7,07 | N 5,61 | Cl 7,10 |
| Gef.: | 67,47 | 7,15 | 5,30 | 7,55 |

$R_f$-Wert: 0,26 (Kieselgel, Methylenchlorid/Methanol = 9/1)

Beispiel 31

2-[N-Methyl-N-((2-methyl-naphth-1-yl)-methyl)-amino-prop-3-yl]-5,6-dimethoxy-phthalimidin

Hergestellt aus 2-[N-Methyl-N-((2-methyl-naphth-1-yl)-methyl)-amino-prop-3-yl]-5,6-dimethoxy-phthalimid und Zink/Eisessig analog Beispiel 8.
Ausbeute: 21 % der Theorie,

| | | | | |
|---|---|---|---|---|
| Ber.: | C 68,63 | H 6,87 | N 6,16 | Cl 7,79 |
| Gef.: | 68,45 | 6,78 | 6,48 | 7,77 |

$R_f$-Wert: 0,52 (Kieselgel, Methylenchlorid/Methanol = 9/1)

Beispiel 32

3-[N-Methyl-N-((naphth-2-yl)-eth-2-yl)-amino-prop-3-yl)-7,8-dimethoxy-1,3,4,5-tetrahydro-2H-3-benzazepin-2-on-hydrochlorid

Hergestellt aus 3-(3-Chlorpropyl)-7,8-dimethoxy-1,3,4,5-tetrahydro-2H-3-benzazepin-2-on und 2-(2-Methylaminoethyl)-naphthalin analog Beispiel 1.
Ausbeute: 23 % der Theorie,
Schmelzpunkt: 215-216 ° C

| | | | | |
|---|---|---|---|---|
| Ber.: | C 69,62 | H 7,30 | N 5,80 | Cl 7,34 |
| Gef.: | 69,43 | 7,45 | 5,63 | 7,96 |

Beispiel I

Tabletten zu 7,5 mg 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

Zusammensetzung:

| 1 Tablette enthält: | |
|---|---|
| Wirksubstanz | 7,5 mg |
| Maisstärke | 59,5 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 ° C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht: 120 mg

Beispiel II

Dragées zu 5 mg 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

| 1 Dragéekern enthält: | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 ° C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel III

Ampullen zu 5 mg 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

| 1 Ampulle enthält: | |
|---|---|
| Wirksubstanz | 5,0 mg |
| Sorbit | 50,0 mg |
| Wasser für Injektionszwecke ad | 2,0 mg |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 10 mg 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

| 1 Zäpfchen enthält: | |
|---|---|
| Wirksubstanz | 0,010 g |
| Hartfett (z.B. Witepsol H 19 und W 45) | 1,690 g |
| | 1,700 g |

Herstellungsverfahren:

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel V

Tropfenlösung mit 10 mg 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on

| 100 ml Lösungen enthalten: | |
|---|---|
| Wirksubstanz | 0,2 g |
| Hydroxyethylcellulose | 0,15 g |
| Weinsäure | 0,1 g |
| Sorbitlösung 70 % Trockensubstanz | 30,0 g |
| Glycerin | 10,0 g |
| Benzoesäure | 0,15 g |
| Dest.Wasser ad | 100 ml |

Herstellungsverfahren:

Dest. Wasser wird auf 70 °C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

**Patentansprüche**

1. Naphthylderivate der allgemeinen Formel

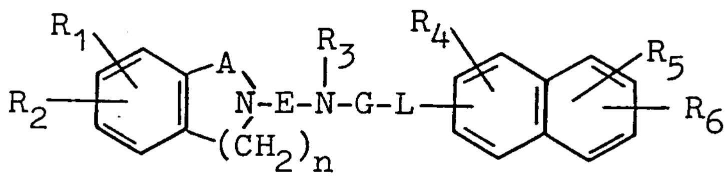

(I)

in der
n die Zahl 1 oder 2,
A eine -CO-,

$$-\underset{x}{C}H_2CO-$$

oder -COCO-Gruppe, wobei das mit x gekennzeichnete Atom mit dem Phenylkern verknüpft ist,
E eine gegebenenfalls durch eine Alkylgruppe mit 1 bis 3 Kohlenstoffatomen substituierte geradkettige Alkylengruppe mit 2 bis 4 Kohlenstoffatomen,
G eine gegebenenfalls durch eine Alkylgruppe mit 1 bis 3 Kohlenstoffatomen substituierte geradkettige Alkylengruppe mit 1 bis 5 Kohlenstoffatomen,
L eine Bindung oder auch ein Sauerstoffatom, wenn G eine gegebenenfalls durch eine Alkylgruppe mit 1 bis 3 Kohlenstoffatomen geradkettige Alkylengruppe mit 2 bis 5 Kohlenstoffatomen darstellt,
$R_1$ und $R_2$, die gleich oder verschieden sein können, Alkyl- oder Alkoxygruppen mit jeweils 1 bis 3 Kohlenstoffatomen in jedem Alkylteil oder $R_1$ und $R_2$ zusammen eine Alkylendioxygruppe mit 1 oder 2 Kohlenstoffatomen,
$R_3$ ein Wasserstoffatom, eine Alkylgruppe mit 1 bis 3 Kohlenstoffatomen oder eine Allylgruppe,
$R_4$, $R_5$ und $R_6$, die gleich oder verschieden sein können, Wasserstoffatome, Alkyl- oder Alkoxygruppen mit jeweils 1 bis 3 Kohlenstoffatomen in jedem Alkylteil bedeuten, deren Enantiomeren und deren Säureadditionssalze.

2. Naphthylderivate der allgemeinen Formel I gemäß Anspruch 1, in der
A, L und n wie im Anspruch 1 definiert sind,
E eine Ethylen- oder n-Propylengruppe,
G eine gegebenenfalls durch eine Methylgruppe substituierte geradkettige Alkylengruppe mit 1 bis 4 Kohlenstoffatomen,
$R_1$ eine Methyl- oder Methoxygruppe,
$R_2$ eine Methyl- oder Methoxygruppe oder $R_1$ und $R_2$ zusammen eine Methylendioxygruppe,
$R_3$ eine Methylgruppe,
$R_4$ ein Wasserstoffatom oder Methylgruppe,
$R_5$ ein Wasserstoffatom, eine Methyl- oder Methoxygruppe,
$R_6$ ein Wasserstoffatom oder eine Methoxygruppe bedeuten, deren Enantiomeren und deren Säureadditionssalze.

3. Naphthylderivate der allgemeinen Formel I gemäß Anspruch 1, in der
n die Zahl 1 oder 2,
A eine -CO- oder $-CH_2CO$-Gruppe,
E eine n-Propylengruppe,
G eine Ethylengruppe,
L eine Bindung,
$R_1$ und $R_2$ jeweils eine Methoxygruppe oder zusammen eine Methylendioxygruppe,
$R_3$ eine Methylgruppe,
$R_4$ ein Wasserstoffatom,
$R_5$ ein Wasserstoffatom, eine Methyl- oder Methoxygruppe und

$R_6$ ein Wasserstoffatom oder eine Methoxygruppe bedeuten, und deren Säureadditionssalze.

**4.** 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-on und dessen Säureadditionssalze.

**5.** 3-[N-Methyl-N-((6-methoxy-5-methyl-naphth-2-yl)-eth-2-yl)-amino-prop-3-yl]-7,8-dimethoxy-1,3,4,5-tetrahydro-2H]-benzazepin-2-on und dessen Säureadditionssalze.

**6.** Physiologisch verträgliche Säureadditionssalze der Verbindungen gemäß den Ansprüchen 1 bis 5 mit anorganischen oder organischen Säuren.

**7.** Arzneimittel, enthaltend eine Verbindung der allgemeinen Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

**8.** Arzneimittel gemäß Anspruch 7 geeignet zur Behandlung von Sinustachykardien und ischämischen Herzerkrankungen.

**9.** Verfahren zur Herstellung eines Arzneimittels gemäß den Ansprüchen 7 und 8, dadurch gekennzeichnet, daß auf nichtchemischem Wege eine Verbindung gemäß den Ansprüchen 1 bis 5 oder dessen physiologisch verträgliches Säureadditionssalz gemäß Anspruch 6 in einen oder mehreren inerten Trägerstoffen und/oder Verdünnungsmitteln eingearbeitet wird.

**10.** Verfahren zur Herstellung von neuen Naphthylderivaten gemäß den Ansprüchen 1 bis 6, dadurch gekennzeichnet, daß

a) e + ine Verbindung der allgemeinen Formel

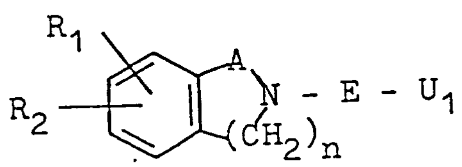

(II)

mit einer + Verbindung der allgemeinen Formel

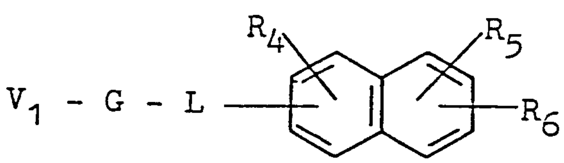

(III)

in denen
$R_1$, $R_2$, $R_4$ bis $R_6$, A, E, G, L und n wie in den Ansprüchen 1 bis 5 definiert sind,
einer der Reste $U_1$ oder V©©$_1$ eine $R_3$©-NH-Gruppe, wobei
$R_3$ wie in den Ansprüchen 1 bis 5 definiert ist und
der andere der Reste $U_1$ oder $V_1$ eine nukleophile Austrittsgruppe wie ein Halogenatom oder eine Sulfonyloxygruppe darstellt, umgesetzt wird oder

b.) eine Verbindung der allgemeinen Formel

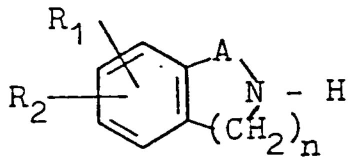

(IV)

in der
$R_1$, $R_2$, A und n wie in den Ansprüchen 1 bis 5 definiert sind, mit einer Verbindung der allgemeinen Formel

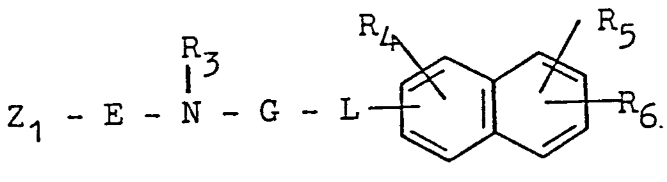

(V)

in der
$R_3$ bis $R_6$, E, G und L wie in den Ansprüchen 1 bis 5 definiert sind und
$Z_1$ eine nukleophile Austrittsgruppe wie ein Halogenatom oder eine Sulfonyloxygruppe darstellt, umgesetzt wird oder
c) eine Verbindung der allgemeinen Formel

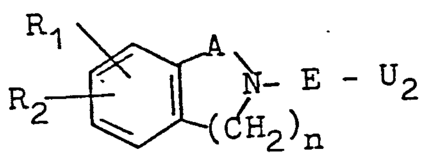

(VI)

in Gegenwart einer Verbindung der allgemeinen Formel

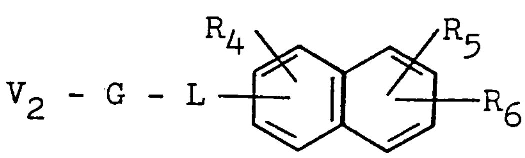

(VII)

in denen
$R_1$, $R_2$, $R_4$ bis $R_6$, A, E, G, L und n wie in den Ansprüchen 1 bis 5 definiert sind,
einer der Reste $U_2$ oder $V_2$ eine $R_3$-NH-Gruppe, wobei
$R_3$ wie in den Ansprüchen 1 bis 5 definiert ist, und
der andere der Reste $U_2$ oder $V_2$ zusammen mit einem Wasserstoffatom des benachbarten Kohlenstoffatoms des Restes G oder E, wobei E und G jeweils wie in den Ansprüchen 1 bis 5 definiert sind, ein Sauerstoffatom bedeutet, reduktiv aminiert wird oder
d) ein Säureamid der allgemeinen Formel

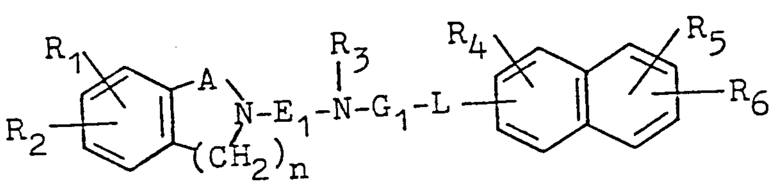

(VIII)

in der
$R_1$ bis $R_6$, A, L und n wie in den Ansprüchen 1 bis 5 definiert sind,
einer der Reste $E_1$ oder $G_1$ die für E oder G in den Ansprüchen 1 bis 5 erwähnten Bedeutungen aufweist und
der andere der Reste $E_1$ oder $G_1$ ebenfalls die für E oder G in den Ansprüchen 1 bis 5 erwähnten Bedeutungen besitzt, wobei jedoch eine zu einem Stickstoffatom benachbarte Methylengruppe durch eine Carbonylgruppe ersetzt sein muß, reduziert wird oder
e) zur Herstellung von Verbindungen der allgemeinen Formel I, in der die -G-L-Gruppe eine gegebenenfalls durch eine Alkylgruppe substituierte Ethylengruppe darstellt, eine Verbindung der allgemeinen Formel

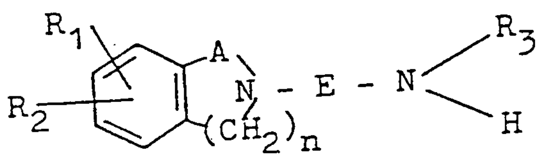

(IX)

in der
$R_1$ bis $R_3$, A, E und n wie in den Ansprüchen 1 bis 5 definiert sind, mit einer Vinylverbindung der allgemeinen Formel

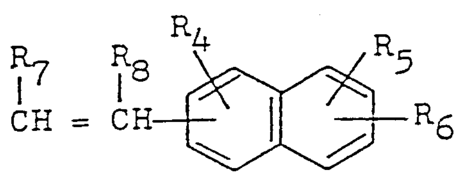

(X)

in der
$R_4$ bis $R_6$ wie in den Ansprüchen 1 bis 5 definiert sind, einer der Reste $R_7$ oder $R_8$ ein Wasserstoffatom und der andere der Reste $R_7$ oder $R_8$ eine Alkylgruppe mit 1 bis 3 Kohlenstoffatomen bedeuten, umgesetzt wird oder
f) zur Herstellung von 2-[N-Methyl-N-((naphth-1-oxy)-eth-2-yl)-amino-prop-3-yl]-5,6-dimethoxy-phthalimidin, 2-[N-Methyl-N-((naphth-2-yl)-methyl)-amino-prop-3-yl]-5,6-dimethoxy-phthalimidin, 2-[N-Methyl-N-((naphth-2-yl-oxy)-but-4-yl)-amino-prop-3-yl]-5,6-dimethoxy-phthalimidin, 2-[N-Methyl-N-((naphth-1-yl)-eth-2-yl)-amino-prop-3-yl]-5,6-dimethoxy-phthalimidin, 2-[N-Methyl-N-((5-methyl-6-methoxy-naphth-2-yl)-eth-2-yl)-amino-prop-3-yl]-5,6-dimethoxy-phthalimidin und 2-[N-Methyl-N-((2-methyl-naphth-1-yl)-methyl)-amino-prop-3-yl]-5,6-dimethoxy-phthalimidin, ein entsprechendes Phthalimid durch 3-stündiges Erhitzen in Zink/Eisessig unter Rückfluß reduziert wird, wobei
erforderlichenfalls ein während den Umsetzungen a) bis e) verwendeter Schutzrest für eine reaktive Gruppe nach der Umsetzung abgespalten wird
und anschließend gewünschtenfalls eine so erhaltene Verbindung der allgemeinen Formel I, welche ein chirales Zentrum enthält, in ihre Enantiomeren aufgetrennt wird und/oder
eine so erhaltene Verbindung der allgemeinen Formel I in ihre Säureadditionssalze, insbesondere in ihre physiologisch verträglichen Säureadditionssalze mit anorganischen oder organischen Säuren übergeführt wird.

## Claims

**1.** Naphthyl derivatives of general formula

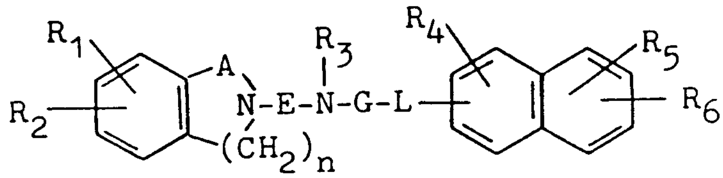

(I)

wherein
n represents the number 1 or 2,
A represents a -CO-,

$$-\underset{x}{C}H_2CO-$$

or
-COCO- group, whilst the atom marked with an x is linked to the phenyl nucleus,
E represents a straight chained $C_{2-4}$ alkylene group optionally substituted by a $C_{1-3}$ alkyl group,
G represents a straight chained $C_{1-5}$ alkylene group optionally substituted by a $C_{1-3}$ alkyl group,
L represents a bond or an oxygen atom, if G represents a straight chained $C_{2-5}$ alkylene group optionally substituted by a $C_{1-3}$ alkyl group,
$R_1$ and $R_2$, which may be identical or different, represent alkyl or alkoxy groups each having 1 to 3 carbon atoms in each alkyl moiety or $R_1$ and $R_2$ together represent an alkylenedioxy group with 1 or 2 carbon atoms,
$R_3$ represents a hydrogen atom, a $C_{1-3}$ alkyl group or an allyl group,
$R_4$, $R_5$ and $R_6$, which may be identical or different, represent hydrogen atoms, alkyl or alkoxy groups each having 1 to 3 carbon atoms in each alkyl moiety, the enantiomers and acid addition salts thereof.

**2.** Naphthyl derivatives of general formula I as claimed in claim 1, wherein
A, L and n are defined as in claim 1,
E represents an ethylene or n-propylene group,
G represents a straight chained $C_{1-4}$ alkylene group optionally substituted by a methyl group,
$R_1$ represents a methyl or methoxy group,
$R_2$ represents a methyl or methoxy group or $R_1$ and $R_2$ together represent a methylenedioxy group,
$R_3$ represents a methyl group,
$R_4$ represents a hydrogen atom or a methyl group,
$R_5$ represents a hydrogen atom, or a methyl or methoxy group,
$R_6$ represents a hydrogen atom or a methoxy group,
and the enantiomers and acid addition salts thereof.

**3.** Naphthyl derivatives of general formula I as claimed in claim 1, wherein
n represents the number 1 or 2,
A represents a -CO- or $-CH_2CO-$ group,
E represents an n-propylene group,
G represents an ethylene group,
L represents a bond,
$R_1$ and $R_2$ each represent a methoxy group or together represent a methylenedioxy group,
$R_3$ represents a methyl group,
$R_4$ represents a hydrogen atom,
$R_5$ represents a hydrogen atom, or a methyl or methoxy group, and
$R_6$ represents a hydrogen atom or a methoxy group,
and the acid addition salts thereof.

**4.** 3-[N-Methyl-N-((6-methoxy-naphth-2-yl)-eth-2-yl)-amino-prop-3-yl]-7,8-dimethoxy-1,3,4,5-tetrahydro-2H-3-benzazepin-2-one and the acid addition salts thereof.

**5.** 3-[N-Methyl-N-((6-methoxy-5-methyl-naphth-2-yl)-eth-2-yl)-amino-prop-3-yl]-7,8-dimethoxy-1,3,4,5-tetrahydro-2H]-benzazepin-2-one and the acid addition salts thereof.

**6.** Physiologically acceptable acid addition salts of the compounds as claimed in claims 1 to 5 with inorganic or organic acids.

**7.** Pharmaceutical composition containing a compound of general formula I as claimed in claims 1 to 5 or a physiologically acceptable acid addition salt thereof as claimed in claim 6 together with one or more inert carriers and/or diluents.

**8.** Pharmaceutical composition as claimed in claim 7 suitable for treating sinus tachycardia and ischaemic heart disease.

**9.** Process for preparing a pharmaceutical composition as claimed in claims 7 and 8, characterised in that a compound as claimed in claims 1 to 5 or a physiologically acceptable acid addition salt thereof as claimed in claim 6 is incorporated, by a non-chemical method, into one or more inert carriers and/or diluents.

**10.** Process for preparing new naphthyl derivatives as claimed in claims 1 to 6, characterised in that

a) a compound of general formula

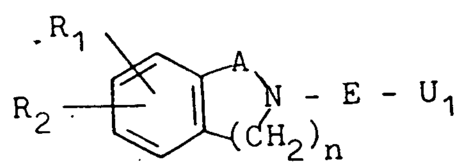

(II)

is reacted with a compound of general formula

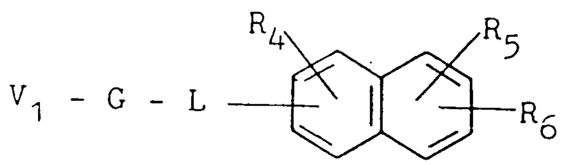

(III)

wherein

$R_1$, $R_2$, $R_4$ to $R_6$, A, E, G, L and n are defined as in claims 1 to 5,

one of the groups $U_1$ or $V_1$ represents an $R_3$-NH- group wherein $R_3$ is defined as in claims 1 to 5, and

the other group $U_1$ or $V_1$ represents a nucleophilically exchangeable group such as a halogen atom or a sulphonyloxy group, or

b) a compound of general formula

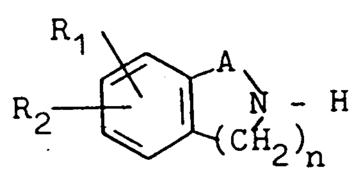

(IV)

wherein

$R_1$, $R_2$, A and n are defined as in claims 1 to 5, is reacted with a compound of general formula

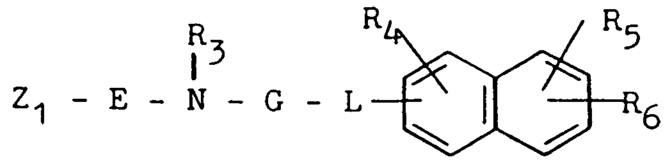

(V)

wherein

$R_3$ to $R_6$, E, G and L are defined as in claims 1 to 5, and

$Z_1$ represents a nucleophilically exchangeable group such as a halogen atom or a sulphonyloxy group, or

c) a compound of general formula

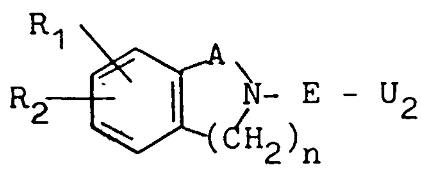

(VI)

is reductively aminated in the presence of a compound of general formula

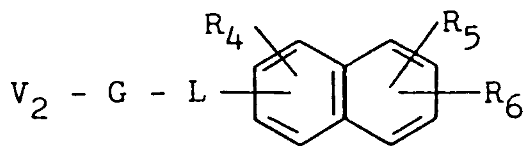

(VII)

wherein

$R_1$, $R_2$, $R_4$ to $R_6$, A, E, G, L and n are defined as in claims 1 to 5,

one of the groups $U_2$ and $V_2$ represents an $R_3$-NH- group wherein $R_3$ is defined as in claims 1 to 5, and

the other group $U_2$ or $V_2$ together with a hydrogen atom of the adjacent carbon atom of the group G or E, wherein E and G are each defined as in claims 1 to 5, represents an oxygen atom, or

d) an acid amide of general formula

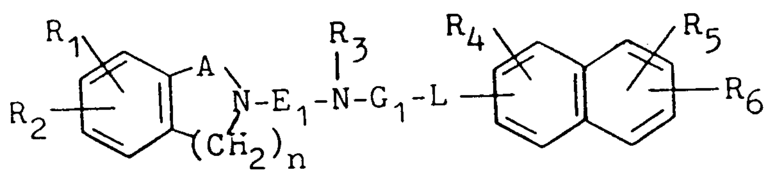

(VIII)

wherein

$R_1$ to $R_6$, A, L and n are defined as in claims 1 to 5,

one of the groups $E_1$ or $G_1$ has the meanings given for E or G in claims 1 to 5, and

the other group $E_1$ or $G_1$ also has the meanings given for E or G in claims 1 to 5,

but a methylene group adjacent to a nitrogen atom must be replaced by a carbonyl group,

is reduced, or

e) in order to prepare compounds of general formula I wherein the -G-L- group represents an ethylene group optionally substituted by an alkyl group,

a compound of general formula

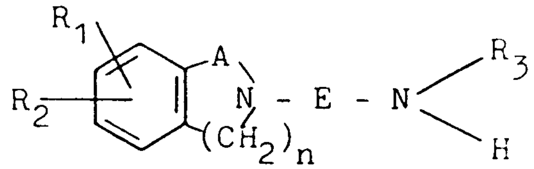

(IX)

wherein
$R_1$ to $R_3$, A, E and n are defined as in claims 1 to 5, is reacted with a vinyl compound of general formula

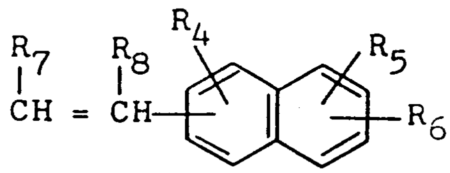

(X)

wherein
$R_4$ to $R_6$ are defined as in claims 1 to 5, one of the groups $R_7$ or $R_8$ represents a hydrogen atom and the other group $R_7$ or $R_8$ represents an alkyl group with 1 to 3 carbon atoms, or
f) in order to prepare 2-[N-methyl-N-((naphth-1-oxy)-eth-2-yl)-amino-prop-3-yl]-5,6-dimethoxy-phthalimidine, 2-[N-methyl-N-((naphth-2-yl)-methyl)-amino-prop-3-yl]-5,6-dimethoxy-phthalimidine, 2-[N-methyl-N-((naphth-2-yl-oxy)but-4-yl)-amino-prop-3-yl]-5,6-dimethoxy-phthalimidine, 2-[N-methyl-N-((naphth-1-yl)-eth-2-yl)-amino-prop-3-yl]-5,6-dimethoxy-phthalimidine, 2-[N-methyl-N-((5-methyl-6-methoxy-naphth-2-yl)-eth-2-yl)-amino-prop-3-yl]-5,6-dimethoxy-phthalimidine and 2-[N-methyl-N-((2-methyl-naphth-1-yl)-methyl)-amino-prop-3-yl]-5,6-dimethoxy-phthalimidine, a corresponding phthalimide is reduced by refluxing for 3 hours in zinc/glacial acetic acid, and if necessary any protecting group used for a reactive group during reactions a) to e) is split off after the reaction,
and subsequently, if desired, a compound of general formula I thus obtained which contains a chiral centre is resolved into its enantiomers, and/or
a compound of general formula I thus obtained is converted into its acid addition salts, particularly its physiologically acceptable acid addition salts with inorganic or organic acids.

## Revendications

1. Dérivés naphtyliques de formule générale

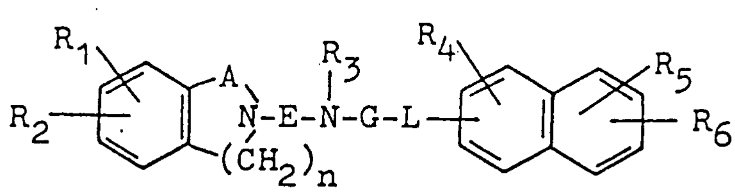

(I)

dans laquelle
n représente le nombre 1 ou 2,
A représente un groupe -CO-,

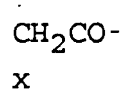

ou -COCO-, l'atome

désigné par x étant relié au noyau phényle,
E représente un groupe alkylène linéaire de 2 à 4 atomes de carbone éventuellement substitué par un groupe alkyle de 1 à 3 atomes de carbone,
G représente un groupe alkylène linéaire de 1 à 5 atomes de carbone éventuellement substitué par un groupe alkyle de 1 à 3 atomes de carbone,
L représente une liaison ou un atome d'oxygène, lorsque G représente un groupe alkylène linéaire de 2 à 5 atomes de carbone éventuellement substitué par un groupe alkyle de 1 à 3 atomes de carbone,
$R_1$ et $R_2$, qui peuvent être identiques ou différents, représentent des groupes alkyle ou alcoxy ayant 1 à 3 atomes de carbone dans chaque partie alkyle ou bien $R_1$ et $R_2$ forment ensemble un groupe alkylènedioxy de 1 ou 2 atomes de carbone,
$R_3$ représente un atome d'hydrogène, un groupe alkyle de 1 à 3 atomes de carbone ou un groupe allyle,
$R_4$, $R_5$ et $R_6$, qui peuvent être identiques ou différents, représentent des atomes d'hydrogène, des groupes alkyle ou alcoxy de 1 à 3 atomes de carbone dans chaque partie alkyle, leurs énantiomères et leurs sels d'addition d'acide.

**2.** Dérivés naphtyliques de formule générale I selon la revendication 1, dans laquelle
A, L et n sont définis comme dans la revendication 1,
E représente un groupe éthylène ou n-propylène,
G représente un groupe alkylène linéaire de 1 à 4 atomes de carbone éventuellement substitué par un groupe méthyle,
$R_1$ représente un groupe méthyle ou méthoxy,
$R_2$ représente un groupe méthyle ou méthoxy ou bien $R_1$ et $R_2$ représentent ensemble un groupe méthylènedioxy,
$R_3$ représente un groupe méthyle,
$R_4$ représente un atome d'hydrogène ou un groupe méthyle,
$R_5$ représente un atome d'hydrogène, un groupe méthyle ou méthoxy,
$R_6$ représente un atome d'hydrogène ou un groupe méthoxy,
leurs énantiomères et leurs sels d'addition d'acide.

**3.** Dérivés naphtyliques de formule générale I selon la revendication 1, dans laquelle
n représente le nombre 1 ou 2,
A représente un groupe -CO ou-$CH_2CO$-,
E représente un groupe n-propylène,
G représente un groupe éthylène,
L représente une liaison,
$R_1$ et $R_2$ représentent chacun un groupe méthoxy ou ensemble un groupe méthylènedioxy,
$R_3$ représente un groupe méthyle,
$R_4$ représente un atome d'hydrogène,
$R_5$ représente un atome d'hydrogène, un groupe méthyle ou méthoxy et
$R_6$ représente un atome d'hydrogène ou un groupe méthoxy, et leurs sels d'addition d'acide.

**4.** 3-[N-méthyl-N-((6-méthoxy-napht-2-yl)-éth-2-yl)-amino-prop-3-yl]-7,8-diméthoxy-1,3,4,5-tétrahydro-2H-3-benzazépin-2-one et ses sels d'addition d'acide.

**5.** 3-[N-méthyl-N-((6-méthoxy-5-méthyl-napht-2-yl)-éth-2-yl)-amino-prop-3-yl]-7,8-diméthoxy-1,3,4,5-tétrahydro-2H]-benzazépin-2-one et ses sels d'addition d'acide.

**6.** Sels d'addition d'acide physiologiquement acceptables des composés selon les revendications 1 à 5 avec des acides inorganiques ou organiques.

**7.** Médicament contenant un composé de formule générale I selon les revendications 1 à 5 ou son sel d'addition d'acide physiologiquement acceptable selon la revendication 6 et un ou plusieurs véhicules et/ou diluants inertes.

**8.** Médicament selon la revendication 7 convenant au traitement des tachycardies sinusales et des maladies cardiaques ischémiques.

**9.** Procédé de préparation d'un médicament selon les revendications 7 et 8, caractérisé en ce que l'on incorpore par voie non chimique un composé selon les revendications 1 à 5 ou son sel d'addition d'acide physiologiquement acceptable selon la revendication 6 dans un ou plusieurs véhicules et/ou diluants inertes.

**10.** Procédé de préparation de nouveaux dérivés naphtyliques selon les revendications 1 à 6, caractérisé en ce que

a) un composé de formule générale

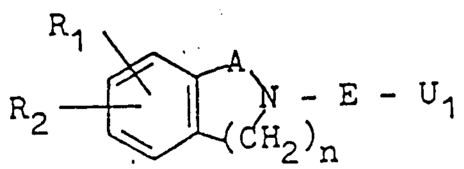

(II)

est mis à réagir avec un composé de formule générale

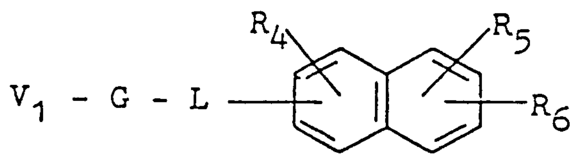

(III)

dans lesquelles

$R_1$, $R_2$, $R_4$ à $R_6$, A, E, G, L et n sont définis comme dans les revendications 1 à 5,

l'un des restes $U_1$ ou $V_1$ représente un groupe $R_3$-NH, $R_3$ étant défini comme dans les revendications 1 à 5, et l'autre des restes $U_1$ ou $V_1$ représente un groupe partant nucléophile tel qu'un atome d'halogène ou un groupe sulfonyloxy, ou bien

b) un composé de formule générale

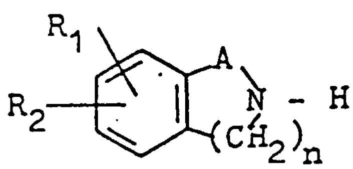

(IV)

dans laquelle

$R_1$, $R_2$, A et n sont définis comme dans les revendications 1 à 5, est mis à réagir avec un composé de formule générale

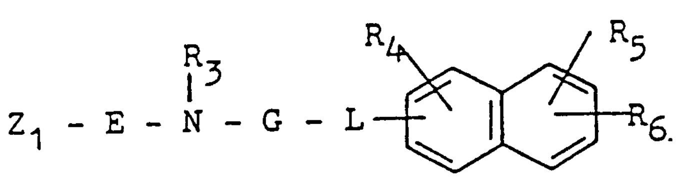

(V)

dans laquelle

$R_3$ à $R_6$, E, G et L sont définis comme dans les revendications 1 à 5 et

$Z_1$ représente un groupe partant nucléophile tel qu'un atome d'halogène ou un groupe sulfonyloxy, ou bien

c) un composé de formule générale

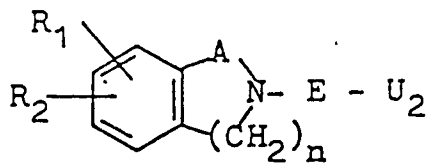

(VI)

est aminé de manière réductrice en présence d'un composé de formule générale

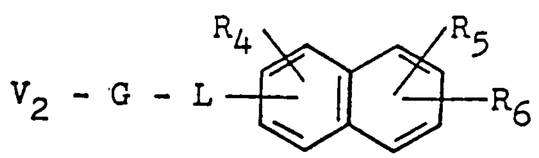

(VII)

dans lesquelles
$R_1$, $R_2$, $R_4$ à $R_6$, A, E, G, L et n sont définis comme dans les revendications 1 à 5,
l'un des restes $U_2$ ou $V_2$ représente un groupe $R_3$-NH, $R_3$ étant défini comme dans les revendication 1 à 5, et l'autre des restes $U_2$ ou $V_2$ représente avec un atome d'hydrogène de l'atome de carbone voisin du reste G ou E un atome d'oxygène, E et G étant définis chacun comme dans les revendications 1 à 5, ou bien
d) un amide d'acide de formule générale

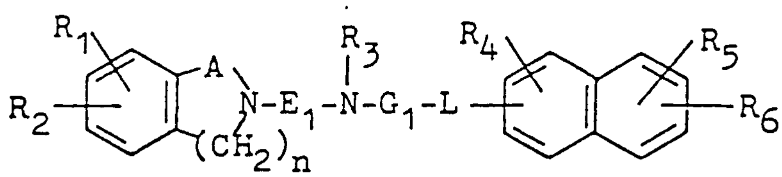

(VIII)

dans laquelle
$R_1$ à $R_6$, A, L et n sont définis comme dans les revendications 1 à 5,
l'un des restes $E_1$ ou $G_1$ at les significations indiquées pour E ou G dans les revendications 1 à 5 et l'autre des restes $E_1$ ou $G_1$ a de même les significations indiquées pour E ou G dans les revendications 1 à 5, un groupe méthylène voisin d'un atome d'azote devant cependant être remplacé par un groupe carbonyle, est réduit ou bien
e) pour la préparation de composés de formule générale I dans laquelle le groupe -G-L- représente un groupe éthylène éventuellement substitué par un groupe alkyle, un composé de formule générale

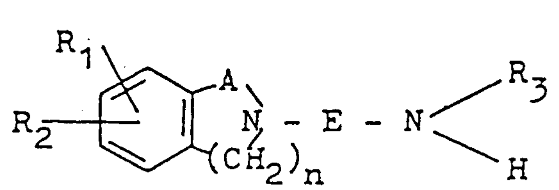

(IX)

dans laquelle
$R_1$ à $R_3$, A, E et n sont définis comme dans les revendications 1 à 5,
est mis à réagir avec un composé vinylique de formule générale

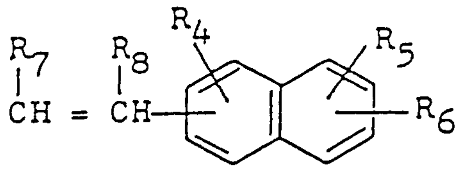

(X)

dans laquelle

$R_4$ à $R_6$ sont définis comme dans les revendications 1 à 5, l'un des restes $R_7$ ou $R_8$ représente un atome d'hydrogène et l'autre des restes $R_7$ ou $R_8$ représente un groupe alkyle de 1 à 3 atomes de carbone, ou bien

f) pour la préparation de la 2-[N-méthyl-N-((napht-1-oxy)-éth-2-yl)-amino-prop-3-yl]-5,6-diméthoxy-phtalimidine, de la 2-[N-méthyl-N-((napht-2-yl)-méthyl)-amino-prop-3-yl]-5,6-diméthoxy-phtalimidine, de la 2-[N-méthyl-N-((napht-2-yl-oxy)-but-4-yl)-amino-prop-3-yl]-5,6-diméthoxy-phtalimidine, de la 2-[N-méthyl-N-((napht-1-yl)-éth-2-yl)-amino-prop-3-yl]-5,6-diméthoxy-phtalimidine, de la 2-[N-méthyl-N-((5-méthyl-6-méthoxy-napht-2-yl)-éth-2-yl)-amino-prop-3-yl]-5,6-diméthoxy-phtalimidine et de la 2-[N-méthyl-N-((2-méthyl-napht-1-yl)-méthyl)-amino-prop-3-yl]-5,6-diméthoxy-phtalimidine, un phtalimide correspondant est réduit par chauffage à reflux pendant 3 heures dans zinc/acide acétique glacial,

si nécessaire un reste protecteur d'un groupe réactif, utilisé pendant les réactions a) à e), est éliminé après la réaction

puis si on le souhaite un composé de formule générale I ainsi obtenu, qui contient un centre chiral, est dédoublé en ses énantiomères et/ou

un composé de formule générale I ainsi obtenu est converti en ses sels d'addition d'acide, en particulier en ses sels d'addition d'acide physiologiquement acceptables avec des acides inorganiques ou organiques.